# FASCICULE DE BREVET EUROPEEN

(11) **EP 1 894 014 B1**
(45) Date de publication et mention de la délivrance du brevet: **21.12.2016**
(21) Numéro de dépôt: 06743634.5
(22) Date de dépôt: 04.04.2006
(51) Int. Cl.: G01N 33/68, C12N 15/62

(54) **METHODE DE SELECTION DANS DES CONDITIONS PHYSICO-CHIMIQUES NON STANDARD DE PROTEINES STABLES**
VERFAHREN ZUR AUSWAHL STABILER PROTEINE IN PHYSIKALISCH-CHEMISCHEN NICHTSTANDARDBEDINGUNGEN
METHOD FOR SELECTING STABLE PROTEINS IN NON-STANDARD PHYSICOCHEMICAL CONDITIONS

(30) Priorité: 10.06.2005 FR 0505935
(43) Date de publication de la demande: 05.03.2008
(73) Titulaire: Biomethodes, 91000 Evry (FR)
(72) Inventeur: DELCOURT, Marc, F-75013 Paris (FR)
(74) Mandataire: Gallois, Valérie
(86) Numéro de dépôt international: PCT/FR2006/000735
(87) Numéro de publication internationale: WO 2006/134240

(56) Documents cités:
- EP-A- 1 097 990
- WO-A-01/29225
- US-A1- 2003 138 843
- US-A1- 2004 078 148
- US-B1- 6 448 087
- WALDO GEOFFREY S ET AL: "Rapid protein-folding assay using green fluorescent protein" BIO/TECHNOLOGY, NATURE PUBLISHING CO. NEW YORK, US, vol. 17, juillet 1999 (1999-07), pages 691-695, XP002182769 ISSN: 0733-222X
- OMOYA K ET AL: "Systematic optimization of active protein expression using GFP as a folding reporter" PROTEIN EXPRESSION AND PURIFICATION, ACADEMIC PRESS, SAN DIEGO, CA, US, vol. 36, no. 2, août 2004 (2004-08), pages 327-332, XP004520299 ISSN: 1046-5928
- NAKAYAMA M ET AL: "A system using convertible vectors for screening soluble recombinant proteins produced in Escherichia coli from randomly fragmented cDNAs" BIOCHEMICAL AND BIOPHYSICAL RESEARCH COMMUNICATIONS, ACADEMIC PRESS INC. ORLANDO, FL, US, vol. 312, no. 3, 19 décembre 2003 (2003-12-19), pages 825-830, XP004474921 ISSN: 0006-291X
- BROUNS STAN J J ET AL: "Engineering a selectable marker for hyperthermophiles" JOURNAL OF BIOLOGICAL CHEMISTRY, vol. 280, no. 12, mars 2005 (2005-03), pages 11422-11431, XP002394096 ISSN: 0021-9258
- LIAO H ET AL: "ISOLATION OF A THERMOSTABLE ENZYME VARIANT BY CLONING AND SELECTION IN A THERMOPHILE", PROCEEDINGS OF THE NATIONAL ACADEMY OF SCIENCES OF USA, NATIONAL ACADEMY OF SCIENCE, WASHINGTON, DC, US, vol. 83, 1 February 1986 (1986-02-01), pages 576-580, XP000971054, ISSN: 0027-8424
- LINDEN A ET AL: "Single-step purification of a recombinant thermostable alpha-amylase after solubilization of the enzyme from insoluble aggregates", JOURNAL OF CHROMATOGRAPHY B : BIOMEDICAL APPLICATIONS, ELSEVIER SCIENCE PUBLISHERS, NL, vol. 737, no. 1-2, 1 January 2000 (2000-01-01), pages 253-259, XP004184274, ISSN: 0378-4347, DOI: 10.1016/S0378-4347(99)00364-3
- Volker Sieber ET AL: "Selecting proteins with improved stability by a phage-based method", Nature Biotechnology, vol. 16, no. 10, 1 October 1998 (1998-10-01), pages 955-960, XP055275357, US ISSN: 1087-0156, DOI: 10.1038/nbt1098-955

## Description

### Introduction.

La présente invention relève du domaine de la biologie moléculaire et plus particulièrement de l'évolution dirigée des protéines.

Le marché annuel des enzymes industrielles et de spécialité est estimé à plusieurs milliards d'euros. Moins de 30 enzymes comptent pour plus de 90% des enzymes industrielles utilisées. La grande majorité est cependant fragile et peu résistante dans les conditions d'usage industriel, d'où la recherche d'enzymes de substitution plus robustes (température, pH, pression). Les enzymes interviennent en complément ou en substitution de méthodes chimiques classiques plus lourdes de manière à rendre ces procédés plus précis et moins coûteux et afin de limiter les externalités négatives sur l'environnement. Beaucoup de ces procédés n'ont pas lieu à pH neutre, température ambiante et pression atmosphérique. Le procédé suivant l'invention permet de rendre des enzymes compatibles avec une utilisation dans des procédés à haute température (50°C à 110°C).

Dans un autre domaine, les protéines et anticorps thérapeutiques constituent une part importante et croissante du marché des médicaments. Un enjeu central, dans l'amélioration d'hormones, enzymes, anticorps ou autres protéines thérapeutiques, est d'augmenter leur stabilité *in vivo.* En effet, si une protéine thérapeutique est rapidement dégradée dans l'organisme, il est plus délicat de réguler la quantité active à un moment donné et il devient nécessaire d'augmenter la fréquence d'administration, ce qui a un coût et une incidence importante sur le confort d'utilisation pour le patient. Le procédé suivant l'invention, parce qu'il permet de sélectionner des protéines stables dans des conditions physico-chimiques non standard, peut permettre indirectement d'augmenter la stabilité *in vivo* de protéines thérapeutiques.

Le cas des hautes températures et des protéines thermostables constitue un cas important de conditions physico-chimiques non standard. Les protéines et peptides thermostables naturels sont issus de micro-organismes poussant à des températures extrêmes. Les micro-organismes thermophiles comprennent notamment : *Bacillus stearothermophilus* (qui vit à une température pouvant atteindre Tmax ∼ 60°C), *Thermus aquaticus* (Tmax ∼ 70°C) et *Thermus thermophilus* (Tmax ∼ 80°C), et les hyper-thermophiles comme *Thermotoga maritime* (Tmax ∼ 90°C) et *Aquifex pyrophilus* (Tmax ∼ 95°C). La capacité de ces organismes à pousser à des températures élevées implique que leurs enzymes et protéines sont stables et actives à ces températures (Moreno R et al. Applied and Environmental Microbiology. 2004; 71:591-93). Ces organismes sont potentiellement sources de diverses enzymes d'intérêt industriel (Vieille C et al. Microbiol. Mol. Biol. Rev. 2001; 65: 1-43; Sterner R et al. Crit. Rev. Biochem. Mol. Biol. 2001. 36:39-106; Pantazaki AA et al. Appl. Microbiol. Biotechnol. 2002; 58:1-12; Niehaus F. et al. Appl. Microbiol. Biotechnol. 1999; 51:711-29).

L'exemple le plus connu d'utilisation de protéines issues d'organismes extrêmophile est le cas des enzymes ADN polymérases thermostables en biologie moléculaire. Grâce à la polymérase thermostable de *Thermus aquaticus* (la *Taq* polymerase), la réaction de polymérisation en chaîne (PCR), qui a révolutionné la biologie moléculaire, a pu être inventée. Depuis, des polymérases thermostables issues d'autres organismes thermophiles, comme la *Pfu* polymerase ou la *Vent* polymerase, ont été produites et commercialisées. En recombinant *in vitro* les séquences de diverses polymérases thermostables et en utilisant une méthode de sélection spécifique pour ces polymérases, une équipe a pu créer de nouvelles polymérases thermostables, à la thermostabilité élevée, moins sensibles à des inhibiteurs courants ou encore capables d'incorporer des nucléotides non standard (Ghadessy FJ et al. Proc Natl Acad Sci USA. 201; 98: 4552-7; Ghadessy FJ et al. Nat Biotechnol. 2004; 22:755-9).

Un autre exemple d'utilisation d'enzyme important issu d'un extrêmophile est le cas de cellulases tirées d'alkaliphiles utilisées comme agent de dégradation de la cellulose dans les détergents (par exemple, la cellulase 103, vendue par Genencor depuis 1997).

Plus généralement, et sans que la liste d' « extremozymes » ci-dessous ne soit exhaustive, les extrêmophiles sont, depuis quelques années, à l'origine de nombreuses nouvelles enzymes (Schiraldi C et al. Trends Biotechnol. 2002 ; 20 :515-20 ; Van den Burg, B. Curr. Op. Microbiol. 2003 ; 6 :213-18). Ainsi, les thermophiles sont la source d'amylases et de glycosidases, utilisées dans le traitement de l'amidon et de divers oses, pour la synthèse d'oligosacharides, de lipases utilisées pour le traitement d'eaux usées ou dans certains détergents, de xylanases utilisées dans l'industrie papetière, de protéases utilisées dans l'agro-alimentaire pour la production d'acides aminés et dans certains détergents. De plus, pouvoir réaliser des procédés industriels à haute température a en outre comme intérêt un risque réduit de contamination microbienne, une viscosité plus faible, des taux de transferts plus élevés et une solubilité accrue des substrats. Les psychrophiles sont la source d'amylases, protéases et lipases utilisées dans des détergents, de déshydrogénases utilisées comme biocapteurs (« biosensor »). Les alkaliphiles sont la source de cellulases et protéases utilisées comme agent de dégradation de polymères dans des formules de détergents ; d'amylases et de lipases utilisées comme additifs alimentaires. Les acidophiles sont la source d'amylases utilisées pour le traitement de l'amidon et d'oxidases utilisées pour la désulfurisation du charbon. Les halophiles sont la source de protéases utilisées en synthèse peptidique et de déshydrogénases, utilisées pour de la biocatalyse en milieu organique (Marhuenda-Egea FC et al. Curr. Op. Biotech. 2002; 13: 385-89). Pouvoir sélectionner des enzymes actives en présence de concentrations élevées de sel est important dans le cas de procédés industriels dans lesquels des enzymes sont utilisés en présence de concentrations élevées de substrats qui se trouvent dans le milieu réactionnel sous forme ionique.

Utiliser directement des bactéries ou archéobactéries extrêmophiles pour isoler des protéines stables et/ou actives dans des conditions physico-chimiques non standard peut donc être efficace. Une telle approche est toutefois rarement possible dans le cas des enzymes issues d'eucaryotes, champignons, levures, plantes ou mammifères : on recense peu d'extrêmophiles eucaryotes (voir néanmoins http://www.nhm.ac.uk/zoology/extreme.html). Il faut alors partir d'une enzyme standard puis l'améliorer, par évolution dirigée, pour la rendre stable et/ou active dans des conditions physico-chimiques non standard. Cette approche d'évolution dirigée de protéines stables dans des conditions physico-chimiques non standard n'est pas limitée aux protéines issues d'eucaryotes, qui sont généralement mésophiles, mais est également intéressante pour toutes les protéines issues d'organismes procaryotes courants, eux aussi mésophiles. Malgré tous les progrès de la biologie moderne, il demeure en effet difficile de déterminer les relations entre la séquence d'une protéine et sa fonction. Il est notamment peu aisé, en pratique, de prévoir précisément quelles mutations permettront de rendre stable une protéine dans des conditions physico-chimiques non standard. L'approche de choix, dans ce contexte où la conception rationnelle est peu opérante, est l'évolution moléculaire dirigée. S'inspirant de l'évolution naturelle Darwinienne, l'évolution dirigée en reproduit, en laboratoire, les principales étapes : obtention d'une diversité génétique, expression des protéines correspondantes puis tri des produits adaptés à des conditions données. Ainsi, l'évolution dirigée commence par obtenir une diversité de gènes puis les exprime, trie les gènes codant des protéines améliorées par rapport à un critère d'intérêt. Plusieurs types de méthodes génériques sont disponibles pour créer de la diversité en modifiant la séquence d'un gène initial : la mutagenèse aléatoire (Leung DW et al. Technique. 1989; 1:11-15; Cadwell RC et al. PCR Methods Appl. 1992; 2:28-33), la mutagenèse dirigée (Kunkel TA et al. J. Methods Enzymol. 1991; 204:125-39; Lacks et al. Methods Enzymol. 1980; 65:138 ; US2004253729), la mutagenèse massive (WO0216606), la mutagenèse par élongation (Matsuura T et al. Nat. Biotechnol. 1999; 17:58-61). D'autres méthodes permettent de créer de la diversité en mélangeant entre elles les séquences de plusieurs gènes : recombinaison *in vitro* par DNA Shuffling (Stemmer WPC. Proc. Natl Acad. Sci. USA. 1993; 91:10747-51; Stemmer WPC. Nature. 1994; 370 : 389-91; US5,605,793; US 5,830,721; US 6,506,603), StEP (US6,153,410; US6,177,263; Zhao et al. Nat. Biotechnol. 1998; 16:258-61; Aguinaldo AM et al. Methods Mol. Biol. 2003; 231:105-10), recombinaison *in vitro* circulaire (demande de brevet FR 0503364 déposée par Biométhodes). Un troisième type de méthode permet enfin d'avoir directement accès à la diversité naturelle sans cultiver les organismes correspondants : le metagénome (Streitt WR et al. Curr Opin Biotechnol. 2004; 15:285-90; Daniel R et al. Curr Opin Biotechnol. 2004; 15:199-204). Toutes ces méthodes d'obtention de diversité sont utiles dans le cadre de la génération, par le procédé selon l'invention, de protéines stables dans des conditions physico-chimiques non standard selon l'invention. On notera que, dans certains cas, les données empiriques et les résultats des modèles limités existants sur les relations séquence-structure-stabilité des protéines, peuvent être intégrés dans le cadre d'une stratégie de mutagenèse. Par exemple, la comparaison des séquences et des structures de protéines avec celles de leurs homologues thermostables montre souvent des différences au niveau des boucles de surface, de la fréquence de résidus hydrophobes avec des chaînes latérales branchées, de la fréquence d'acides aminés chargés ou de la fréquence de ponts disulfures et il est possible d'intégrer ce type de résultat au moment du choix des cibles de la mutagenèse et du type de substitution à opérer.

Après l'obtention de diversité au niveau des polynucléotides codant, la deuxième étape de l'évolution dirigée, le tri, a lieu au niveau de leurs produits protéiques et peut se faire suivant deux modalités : le criblage (ou screening, notamment le criblage haut-débit ou « high-throughput screening ») et la sélection. Le criblage consiste à trier, une par une, les protéines et les gènes correspondants sur la base d'un test réalisé typiquement dans des puits de microplaques à 96, 384 ou 1536 puits. Suivant le degré d'automatisation, on peut ainsi trier de l'ordre de 10³ à 10⁵ variants par jour. Les méthodes dites de sélection sont plus proches de la sélection naturelle, en ce qu'elles trient « en masse », en parallèle, tous les variants répondant à un critère donné, et les isolent de la majorité des variants, non améliorés. On peut ainsi trier de l'ordre de 10⁶ et jusqu'à 10¹³ variants simultanément. On voit aisément que, lorsqu'elles sont disponibles, les méthodes de sélection permettent de trier beaucoup plus facilement une diversité beaucoup plus grande que les méthodes de criblage. Des méthodes de sélection comme le phage display ou le ribosome display, sont disponibles lorsque le critère d'intérêt est l'affinité de la protéine pour un ligand donné, et ont été employées avec succès dans le domaine des anticorps. Ces méthodes ont pu être adaptées à des cas de sélection pour l'activité enzymatique ou la stabilité dans des conditions standard (Amstutz P et al. J. Am. Chem. Soc. 2002; 124:9396-9403; Jung S et al. J. Mol. Biol. 1999; 294:163-180.5). Toutefois, dans le domaine de la stabilité et de l'activité dans des conditions physico-chimiques non standard, les méthodes de criblage individuel restent prépondérantes et on ne dispose pas de méthode de sélection universelle, indépendante de la fonction de la protéine d'intérêt, si ce n'est la méthode décrite dans le document par Sieber V et al (1998), Nature Biotechnology, vol.16, pp.955-960. Comme on vient de le voir, ces méthodes de criblage sont de mise en oeuvre assez lourde, nécessitant une mécanisation assez importante et ayant un coût élevé, pour des tailles de banques limitées. Disposer d'une méthode universelle de sélection de protéines stables dans des conditions physico-chimiques non standard permettrait ainsi un saut qualitatif dans le domaine de l'évolution dirigée des protéines, et notamment des enzymes industrielles, des protéines thérapeutiques, des biocapteurs et du diagnostic. L'application d'une telle méthode, conjointement aux méthodes de création de diversité génétique existantes, permettrait l'obtention d'un grand nombre de nouvelles protéines très diverses, d'intérêt industriel ou thérapeutique majeur par évolution dirigée. C'est précisément l'objet du procédé selon l'invention.

### Résumé de l'invention.

La présente invention dans son sens le plus étendu est telle que définie dans les revendications indépendantes.

Le procédé selon l'invention est tel que défini dans les revendications et concerne une méthode de sélection de protéines stables dans des conditions physico-chimiques non standard de température, caractérisé par l'expression, dans un micro-organisme extrêmophile, de variants de la protéine d'intérêt sous forme de protéine de fusion avec une protéine rapporteur *Π* stable dans les conditions non standard considérées jouant le rôle de marqueur de sélection.

Ainsi, la présente invention concerne un procédé de sélection de protéines stables, en particulier dans des conditions physico-chimiques non standard, caractérisé en ce qu'une banque de variants d'une protéine d'intérêt est exprimée dans un micro-organisme extrêmophile sous la forme d'une protéine de fusion avec une protéine rapporteur stable dans des conditions physico-chimiques non standard, et en ce que un ou plusieurs variants stables de la protéine d'intérêt sont sélectionnés à partir des micro-organismes dans lesquels la protéine rapporteur est active.

Le micro-organisme extrêmophile peut être une bactérie, une archéo-bactérie, ou un eucaryote extrêmophile. Le micro-organisme est un micro-organisme thermophile ou hyper-thermophile. En particulier, le micro-organisme thermophile ou hyper-thermophile peut être sélectionné parmi les micro-organismes thermophiles ou hyper-thermophiles suivant : *Thermus aquaticus, Thermus thermophilus, Thermotoga maritime* ou *Aquifex pyrophilus.*

De préférence, la protéine rapporteur stable est une protéine dont l'expression correcte permet la survie du micro-organisme et la sélection du variant stable de la protéine d'intérêt est opérée par survie du micro-organisme. Dans un mode de réalisation préféré, la protéine rapporteur est une protéine conférant la résistance à un antibiotique. Par exemple, la protéine rapporteur peut être une version thermostable d'une protéine de résistance à un antibiotique. De préférence, la protéine rapporteur est une version thermostable d'une protéine de résistance à la kanamycine ou à la bléomycine. Dans un mode de réalisation encore plus préféré, la protéine rapporteur est une kanamycine nucleotidyltransférase thermostable. Dans un mode de réalisation alternatif, la protéine rapporteur est un marqueur d'auxotrophie.

Dans un autre mode de réalisation, la protéine rapporteur est une protéine dont l'expression correcte conduit directement ou indirectement à la transformation d'un substrat en un produit, le produit et le substrat différant par leurs couleurs, et la sélection du variant stable de la protéine d'intérêt est opérée sur la base de la couleur des micro-organismes. Par exemple, la protéine rapporteur peut être une version stable dans des conditions physico-chimiques non standard de la béta-galactosidase.

Dans un mode de réalisation supplémentaire, la protéine rapporteur est fluorescente ou luminescente et la sélection du variant stable de la protéine d'intérêt est opérée par détection de la fluorescence ou luminescence. La protéine rapporteur peut ne pas être fluorescente ou luminescente dans les conditions physico-chimiques non standard où elle est produite mais devenir fluorescente lorsqu'on la place dans des conditions physico-chimiques standard. De préférence, la protéine rapporteur est la GFP (green fluorescent protein) ou un variant de la GFP. Dans un mode de réalisation préféré, la sélection est effectuée en utilisant un FACS ou par simple sélection visuelle de colonies bactériennes.

Dans un mode de réalisation préféré, la protéine d'intérêt peut être située en N-terminal de la protéine rapporteur dans la protéine de fusion. Dans un mode de réalisation alternatif, la protéine d'intérêt est située en C-terminal de la protéine rapporteur dans la protéine de fusion. Facultativement, la protéine de fusion comprend un espaceur peptidique.

La banque de variants de la protéine d'intérêt peut être générée par une technique de mutagenèse connue de l'homme du métier, de préférence sélectionnée parmi le groupe suivant : la technologie Massive Mutagenesis^{®}, la mutagenèse aléatoire, la mutagenèse dirigée, la mutagenèse à saturation, la mutagenèse par élongation, et la mutagenèse *in vivo,* une méthode de recombinaison génétique *in vivo* ou *in vitro,* ou une combinaison de celles-ci. Dans un mode de réalisation préféré, elle est créée par la technologie Massive Mutagenesis^{®}. Dans un mode de réalisation préféré alternatif, elle est créée par recombinaison *in vitro* circulaire de plusieurs gènes naturels ou synthétiques codant la protéine d'intérêt.

La banque de variants de la protéine d'intérêt peut également être créée par clonage direct d'acides nucléiques tirés d'une source environnementale par utilisation des techniques du metagénome.

Dans un mode de réalisation préféré, le micro-organisme est thermophile ou hyper-thermophile, la protéine rapporteur est une protéine thermostable conférant la résistance à un antibiotique, la protéine d'intérêt est située en N-terminal de la protéine rapporteur dans la protéine de fusion, et la banque de variants de la protéine d'intérêt est créée par la technologie Massive Mutagenesis^{®}. De préférence, le micro-organisme est T. Thermophilus, la protéine rapporteur est une kanamycine nucleotidyltransférase thermostable, et la protéine de fusion comprend un espaceur peptidique.

Le présent document décrit les protéines ayant une stabilité accrue dans des conditions physico-chimiques non standard sélectionnées selon le procédé de la présente invention. Notamment, sont considérés les enzymes, anticorps, hormones, cytokines ou autres protéines thérapeutiques sélectionnées selon le procédé de la présente invention et qui présentent une stabilité *in vivo* accrue.

La présente invention concerne également une protéine de fusion comprenant une protéine d'intérêt ou un variant de celle-ci et une version thermostable d'une protéine conférant la résistance à un antibiotique. De préférence, la protéine conférant la résistance à un antibiotique est la kanamycine nucleotidyltransférase ou la protéine de résistance à la bléomycine. L'invention concerne en outre un acide nucléique ou vecteur d'expression codant une protéine de fusion selon la présente invention. De préférence, le vecteur comprend les éléments nécessaires à la réplication dans un micro-organisme extrêmophile. Facultativement, le vecteur peut comprendre en outre une origine de réplication pour un autre micro-organisme couramment utilisé en laboratoire, par exemple *E. Coli,* et/ou un autre gène de résistance à un antibiotique, par exemple un gène de résistance à l'ampicilline.

### Description détaillée de l'invention.

Le procédé selon l'invention permet la sélection, au sein d'un ensemble de variants d'une protéine d'intérêt, de variants présentant une stabilité accrue dans des conditions physico-chimiques non standard. Ces conditions physico-chimiques non standard ou « extrêmes » sont ici définies par une température élevée (50°C à 110°C).

Cette stabilité dans des milieux plus ou moins extrêmes peut être intéressante et recherchée en soi. Dans de nombreux cas, la sélection pour une stabilité accrue dans des conditions physico-chimiques non standard pourra en outre conduire indirectement à améliorer les caractéristiques de la protéine dans d'autres conditions non standard, ou dans des conditions standard. Ainsi, il a été montré qu'un variant ayant acquis de la thermostabilité peut aussi avoir acquis de la résistance aux solvants (Liu *et al,* 2006 ; Hao *et al,* 2004), aux détergents (Liao, 1993), à la protéolyse (Mukherjee *et al,* 2005 ; Amin *et al,* 2004). La thermostabilité est également associée à l'accroissement de la demi-vie en conditions douces (Hao *et al,* 2004 ; Wintrode *et al,* 2001). Le procédé selon l'invention permet donc l'amélioration de la stabilité, au sens large, de protéines.

Plus précisément, partant d'un gène *G₀* codant la protéine d'intérêt *P₀* que l'on souhaite améliorer, le procédé selon l'invention comprend les trois étapes suivantes :
a) A partir du gène d'intérêt *G₀*, préparation d'une banque de variants *Gᵢ* codant les variants protéiques *Pᵢ* (où *i*=1, 2... *N* avec *N* compris entre 2 ou 10 et 10¹⁰, et de préférence compris entre 10³ et 10⁸) dans un vecteur de telle sorte que des protéines *Pᵢ* soient exprimées avec une protéine rapporteur *Π* sous la forme de protéines de fusion *Pᵢ-Π.*
b) transformation de la banque de vecteurs obtenue lors de l'étape a) dans un organisme extrêmophile et expression des protéines de fusion *Pᵢ-Π* dans des conditions physico-chimiques non standard données, qui sont compatibles avec les conditions de vie de l'organisme extrêmophile.
c) sélection, sur la base de l'activité de *Π,* de clones exprimant la protéine de fusion *Pᵢ-Π* correctement.

Dans un premier mode de réalisation, la préparation de la banque de variants de l'étape a) comprend les étapes suivantes : on prépare une construction ou un vecteur comprenant le gène *G₀* codant la protéine d'intérêt *P₀* et la séquence codant une protéine rapporteur *Π* sous forme d'une protéine de fusion *Pₒ-Π,* puis la banque de variants *Gᵢ* (où *i*=1, 2... *N* avec *N* compris entre 2 ou 10 et 10¹⁰, et de préférence compris entre 10³ et 10⁸) est généré à partir de cette construction ou de ce vecteur.

Dans un mode de réalisation alternatif, la préparation de la banque de variants de l'étape a) comprend les étapes suivantes : construction d'une banque de variants *Gᵢ* (où *i*=1, 2... *N* avec *N* compris entre 2 ou 10 et 10¹⁰, et de préférence compris entre 10³ et 10⁸), puis les gènes *Gᵢ* codant les variants protéiques *Pᵢ* (*i*=1, 2... *N*) sont clonés dans un vecteur de telle sorte que des protéines *Pᵢ* soient exprimées avec une protéine rapporteur *Π*, stable dans des conditions physico-chimiques non standard, sous la forme de protéines de fusion *Pᵢ-Π.*

Dans un mode de réalisation particulier, le critère d'amélioration retenu est la thermostabilité, l'extrêmophile est un thermophile et, partant d'un gène *G₀* codant la protéine *P₀* qu'on souhaite améliorer, le procédé selon l'invention est caractérisé par la succession des trois étapes suivantes :
a) A partir du gène d'intérêt *G₀*, préparation d'une banque de variants *Gᵢ* codant les variants protéiques *Pᵢ* (où *i*=1, 2... *N* avec *N* compris entre 2 ou 10 et 10¹⁰, et de préférence compris entre 10³ et 10⁸) dans un vecteur de telle sorte que des protéines *Pᵢ* soient exprimées avec une protéine rapporteur *Π* sous la forme de protéines de fusion *Pᵢ-Π.*
b) transformation de la banque de vecteurs obtenue lors de l'étape a) dans un organisme thermophile et expression à haute température des protéines de fusion *Pᵢ-Π.*
c) sélection, sur la base de l'activité de *Π* à haute température, de clones exprimant la protéine de fusion *Pᵢ-Π* correctement.

Une approche relativement similaire est décrite dans la littérature dans le cas de l'amélioration de la solubilité des protéines insolubles : dans ces exemples, des banques de mutants sont fusionnés en phase avec la GFP, et les mutants ayant acquis une certaine solubilité sont sélectionnés sur le critère de leur fluorescence plus importante (brevets américains 6,867,042 et 6,448,087 ; demandes de brevets américains 20030138843 et 20040078148).

La présente invention diffère de façon importante en ce que l'on ne recherche pas ici une amélioration de la solubilité des protéines, mais une amélioration de la stabilité dans des conditions extrêmes. A ce titre, les hôtes de sélection utilisés sont des organismes extrêmophiles qui ne sont pas mentionnés dans les documents cités précédemment. En outre, la méthode nécessite la conception de vecteurs navettes adaptés aux organismes extrêmophiles, mais de préférence également adaptés à un organisme standard comme *E*. *Coli.* Le gène de fusion doit correspondre aux conditions non standard choisies. Ainsi, le gène rapporteur *Π* doit être stable dans les conditions physico-chimiques non standard considérés. En effet, le procédé selon l'invention s'applique à l'amélioration de la thermostabilité. Si optimiser une protéine pour qu'elle soit stable et/ou active dans ces conditions peut conduire à améliorer sa solubilité, l'inverse n'est en général pas vrai. Ainsi, de nombreuses protéines sont exprimées très facilement de manière hétérologue chez *E. Coli* et sont donc très solubles sans pour autant être stables dans des conditions physico-chimiques non standard, notamment sans être thermostables. Par ailleurs, les effets sur la séquence d'une amélioration de l'activité à basse température et à haute température sont évidemment différents. On peut encore illustrer cette différence en considérant le cas de protéines qui sont très thermostables, mais peu solubles (voir par exemple: Linden A. et al. J Chromatogr B Biomed Sci Appl. 2000 Jan 14;737(1-2):253-9). Une analyse des déterminants structuraux de la thermostabilité montre en fait que celle-ci est liée à une meilleure solubilité mais également à une conformation plus compacte (Gromiha MM et al. Biophys Chem. 1999; 82:51-67).

Le détail des mécanismes assurant le couplage entre la stabilité des deux partenaires d'une protéine de fusion n'est pas bien élucidé. Néanmoins, il a été observé que lorsqu'on fusionne une protéine d'intérêt à une protéine rapporteur, la conformation de l'une et de l'autre sont liées, avec généralement un ordre : une conformation correcte de la protéine située du côté COOH est dépendante d'une conformation également correcte de la protéine localisée à l'extrémité NH₂. Ces effets de conformation lors de la synthèse sont parfois décrits sous le terme de « nucléation ». Dans des cas plus rares, cependant, c'est l'inverse qui est observé si bien qu'une approche empirique consistant à tester les deux orientations est nécessaire pour déterminer le sens de la causalité.

Les protéines *Pᵢ* stables dans des conditions physico-chimiques non standard sélectionnées peuvent, en même temps qu'elles ont augmenté leur stabilité avoir diminué leur activité (au sens d'activité enzymatique, ou d'affinité, dans le cas des anticorps, par exemple). Parfois, on souhaite obtenir des protéines qui sont non seulement stables mais également actives dans des conditions physico-chimiques non standard. Dans ce cas, en aval de la méthode générique de sélection pour la stabilité offerte par le procédé selon l'invention, un criblage (ou une sélection) secondaire des clones améliorés sur le critère de leur activité est nécessaire. Ce criblage secondaire peut être effectué directement sur la protéine *Pi* fusionnée au gène rapporteur. Alternativement, il peut être nécessaire à ce stade de transposer les mutations identifiées sur les protéines *Pi* stables dans un autre vecteur, éventuellement adapté à l'expression dans un autre hôte (cellules mammifères, levures, ou autres bactéries par exemple), par sous-clonage ou par mutagenèse dirigée. Cette transposition est en particulier souhaitable lorsque la fusion à un gène rapporteur affecte l'activité de la protéine d'intérêt.

Lorsque, dans des conditions physico-chimiques non standard données, une protéine *Pᵢ* n'est pas stable, la protéine de fusion *Pi-Π* ne sera pas stable. En sélectionnant au sein de la banque celles des protéines de fusion *Pᵢ-Π* qui correspondent à une protéine *Π* stable, (ou, suivant les cas, actives) le procédé selon l'invention permet de sélectionner les protéines *Pᵢ* stables dans ces conditions physico-chimiques non standard données.

La première contrainte évidente pour la protéine *Π* est qu'elle doit être stable dans les conditions de sélection. Par exemple, si on veut sélectionner une protéine *Pᵢ* stable à 80 °C, il faut que la protéine *Π* soit elle-même stable à une température de 80 °C ou plus. Pour que la protéine thermostable Π puisse jouer son rôle de marqueur de sélection, il est en outre nécessaire qu'on puisse de manière simple isoler des clones exprimant correctement cette protéine au sein de clones qui ne l'expriment pas.

La solution la plus simple est une sélection par survie : lorsque la protéine *Π* est active, l'organisme extrêmophile dans lequel a lieu la sélection survit, dans le cas contraire, il meurt. Si la protéine *Pᵢ* est stable dans les conditions physico-chimiques non standard choisies, alors la protéine de fusion *Pᵢ- Π* s'exprime correctement à l'étape b), ce qui permet à *Π* d'être active et aux cellules de survivre. De sorte qu'à l'étape c), la sélection consiste simplement à récupérer les cellules ayant poussé.

Une méthode simple dans ce contexte est d'utiliser une protéine de résistance à un antibiotique. Lors de l'étape c), la sélection s'effectue en faisant pousser, en milieu liquide ou en milieu solide, les cellules du micro-organisme extrêmophile utilisé en présence de l'antibiotique correspondant. Seules les cellules exprimant correctement, dans les conditions physico-chimiques non standard choisies, le gène de résistance à cet antibiotique pousseront.

Par exemple, si la propriété recherchée est la thermostabilité, un antibiotique pour lequel l'existence de variants thermostables de l'enzyme de résistance a été rapportée est la kanamycine. La kanamycine est un antibiotique peu coûteux et couramment utilisé dans les laboratoires de recherche. Il s'agit d'un bactéricide puissant, agissant indépendamment de la densité bactérienne, et ayant un effet à la fois très intense et très rapide *in vitro.* La kanamycine nucleotidyltransférase (KNTase) est une enzyme permettant aux bactéries de résister à l'antibiotique. Cette enzyme a été isolée à partir de *Staphylococcus aureus.* Elle catalyse le transfert d'un nucléoside monophosphate d'un nucléotide vers le groupement hydroxyle en 4' de la kanamycine. Ceci a pour effet de neutraliser l'effet de l'antibiotique. Il existe différents mutants de l'enzyme permettant la résistance qui sont stables et actives à des températures allant jusqu'à plus de 70 °C (Liao HH Enzyme Microb. Technol. 1993; 15:286-92; Sakon J et al. Biochemistry. 1993; 32: 11977-84; Liao HH et al. Proc. Natl. Acad. Sci. USA. 1986 ; 83:576-580). Les séquences nucléotidique et en acides aminés d'une kanamycine nucleotidyltransférase thermostable sont par exemple les séquences décrites dans SEQ ID Nos 1 et 2. D'autres gènes de résistance à la kanamycine thermostables ont également été décrits (Hoseki J et al. J. Biochem. 1999 ; 126:951-6). On notera que la kanamycine elle-même est stable à ces températures élevées. Les variants d'une protéine d'intérêt qui sont thermostables et sont fusionnés à la KNTase thermostable permettent à l'organisme thermophile de survivre à haute température en présence de kanamycine. A l'inverse, des variants non thermostables de la protéine d'intérêt empêchent à la KNTase thermostable d'être exprimée correctement et l'organisme thermophile meurt. Cette sélection directe par survie est remarquable en ce qu'on obtient une discrimination très nette entre les différents variants de la protéine, ce qui permet la sélection directe des clones exprimant les variants thermostables de la protéine d'intérêt.

Il est aujourd'hui possible de fabriquer d'autres protéines conférant la résistance à un antibiotique à des micro-organismes (bactéries ou archéobactéries) extrêmophiles, et en particulier thermophiles. Par exemple, en faisant évoluer la protéine équivalente chez la bactérie mésophile *Streptoalloteichus hindustanus,* un groupe vient récemment d'obtenir une protéine conférant la résistance à la bléomycine à la bactérie thermophile *Thermus thermophilus* HB27 (Brouns et al. J. Biol. Chem. 2005; 280:11422-31). Les séquences nucléotidique et en acides aminés d'une protéine conférant la résistance à la bléomycine thermostable sont par exemple les séquences décrites dans SEQ ID Nos 3 et 4.

Par des méthodes de sélection évidentes pour l'homme du métier, il est plus généralement possible de fabriquer des protéines conférant la résistance à un antibiotique à des organismes extrêmophiles variés. On clone par exemple une banque de mutants du gène de résistance qu'on veut adapter aux conditions de vie extrêmes de l'organisme d'intérêt, on transforme le micro-organisme extrêmophile avec cette banque et les cellules de ce micro-organisme qui pousseront en présence de l'antibiotique seront porteuses de variants stables et actifs dans les conditions de vie de cet organisme. Par exemple, l'article de Brouns et collaborateurs (Brouns *et al.* supra) illustre une méthode simple permettant d'obtenir, par évolution dirigée, un gène conférant la résistance à haute température à un antibiotique. Il est ainsi possible d'obtenir de manière simple une gamme de protéines *Π* grâce à laquelle le procédé suivant l'invention permettra de sélectionner des protéines stables dans des conditions physico-chimiques non standard variées.

Dans un mode de réalisation particulier, alternativement à l'utilisation d'antibiotiques, la sélection par survie est réalisée en utilisant des souches auxotrophes. Ces souches ont été modifiées par délétion d'un gène, la délétion étant létale dans des conditions de pousse sur milieu minimum. En complémentant ces souches avec un vecteur porteur du marqueur d'auxotrophie, les souches qui expriment correctement ce gène survivent. Le marqueur d'auxotrophie permet de complémenter le gène supprimé et de permettre à la cellule de survivre en milieu minimum. Dans un mode de réalisation particulier, le procédé suivant l'invention utilise ce principe et un vecteur exprimant en fusion le marqueur d'auxotrophie et la banque de variants de gènes codant la protéine d'intérêt que l'on souhaite améliorer sera utilisé. Ainsi, si la protéine de fusion est correctement exprimée, elle complémente le gène supprimé et permet à la cellule de survivre lorsque celle-ci est cultivée sur un milieu non complémentant. Dans le cas contraire, la cellule est incapable de survivre.

La sélection peut être effectuée en utilisant un critère autre que la survie. Tout système de protéine rapporteur stable dans des conditions physico-chimiques non standard connu de l'homme du métier peut être utilisé. Dans un mode de réalisation, la protéine *Π* est un mutant stable dans des conditions physico-chimiques non standard d'une protéine qui est fluorescente ou luminescente, par exemple la GFP (green fluorescent protein). Plusieurs mutants supportant des températures un peu plus élevées que la GFP sauvage sont aujourd'hui disponibles, et possèdent des caractéristiques spectrales variées (voir par exemple : Siemering KR et al. Curr Biol. 1996; 6:1653-63). Des variants GFP ou d'autres protéines fluorescentes ou luminescentes ou des enzymes dont le produit est fluorescent qui sont stables et/ou actives dans des conditions physico-chimiques non standard peuvent de manière analogue être utilisés dans le procédé suivant l'invention.

Dans un mode de réalisation particulier, la protéine rapporteur est stable et éventuellement fluorescente dans les conditions physico-chimiques non standard choisies et elle est fluorescente ou luminescente dans des conditions standard. Notamment, la protéine rapporteur utilisée peut ne pas être fluorescente ou luminescente dans les conditions physico-chimiques non standard où elle est produite mais elle devient fluorescente lorsqu'on la place dans des conditions physico-chimiques standard. Par exemple, elle peut être produite à haute température, subir une dénaturation réversible et retrouver une conformation la rendant fluorescente ou luminescente lorsqu'elle est placée à température ambiante.

Les cellules contenant des protéines *Pᵢ* stables dans des conditions physico-chimiques non-standard expriment correctement la protéine de fusion *Pᵢ- Π* et ces cellules fluorescent lorsqu'elles sont excitées à une longueur d'onde adaptée. Ces cellules peuvent ensuite être triées de manière simple et rapide, dans des conditions standard (notamment : à température ambiante) en utilisant un FACS (« fluorescence activated cell sorter »). Il est à noter que, dans ce mode de réalisation, il n'est absolument pas nécessaire, pour que la sélection ait lieu correctement, que la protéine fluoresce dans les conditions physico-chimiques non standard dans lesquelles pousse l'organisme extrêmophile choisi et dans lesquelles la protéine de fusion *Pᵢ- Π* est exprimée. Il suffit, en effet, que la protéine rapporteur soit stable, ou au pire subisse une dénaturation réversible, dans les conditions physico-chimiques non standard choisies, puisque c'est son activité dans des conditions standard (notamment : à température ambiante) qui permettra ensuite la sélection.

Dans un mode de réalisation particulier, les cellules de micro-organismes extrêmophiles exprimant les protéines de fusion *Pᵢ- Π* sont cultivées sur milieu solide et la sélection se fait simplement en prélevant les colonies fluorescentes ou luminescentes.

Dans un autre mode de réalisation particulier, la protéine *Π* est une protéine qui, directement ou indirectement, conduit à la transformation d'un substrat en un produit coloré. La sélection a alors lieu de préférence sur milieu solide en prélevant les colonies de la couleur du produit coloré. La protéine rapporteur est de préférence un variant stable de la béta-galactosidase.

Une autre protéine rapporteur thermostable utilisable dans la présente invention est l'esterase thermostable de A. *acidocaldarius* (Agafonov DE et al. FEBS Lett. 2005;579:2082-86.). Cet article décrit en outre un test permettant de détecter facilement cette protéine.

Il est probable que de nombreuses autres protéines pouvant être utilisées comme marqueur de sélection chez des organismes extrêmophiles vont être fabriquées dans les années qui viennent. Le procédé suivant l'invention n'est pas dépendant d'une protéine *Π* particulière.

Quelle que soit la nature de la protéine *Π*, lorsqu'on construit le gène codant la protéine de fusion *Pᵢ*-*Π*, un certain nombre de règles classiques sont à respecter. Ainsi, la liaison entre le gène d'intérêt et celui codant pour la protéine rapporteur doit se faire sans interrompre la phase de lecture. Il est également nécessaire de prendre soin aux contraintes stériques de l'une et l'autre protéine. En effet, il est possible que la fusion de la protéine d'intérêt et de la protéine rapporteur empêche l'une ou l'autre d'adopter sa conformation naturelle. Sa structure en serait modifiée, et son activité annulée. Afin d'éviter ce possible écueil, il est généralement souhaitable d'introduire une séquence codant un peptide de liaison (« linker ») entre le gène codant pour la protéine d'intérêt et celui codant pour la protéine rapporteur. Ce peptide de liaison a en général une structure la plus neutre possible (ie : généralement sans aucun motif de structure secondaire), et ne présente pas en lui-même d'activité particulière. Il est typiquement composé de 8 ou 9 acides aminés neutres qui ne doivent pas influer sur la structure des protéines et ne doivent pas être la cible des protéases. Il est préférable qu'il soit flexible et hydrophile (Sieber V. Methods Mol Biol. 2003 ; 230:45-55; US6,448,087). Les acides aminés formant le peptide de liaison sont de préférence sélectionnés dans la groupe consistant en Gly, Ser, Ala, Val et Thr. De manière classique, les peptides de liaison sont souvent formés par un motif du type (Gly₄-Ser)ₓ, avec x = 3 par exemple. Le fragment codant le peptide de liaison doit bien entendu être introduit de telle sorte que la phase de lecture soit conservée sur l'ensemble constitué par la protéine d'intérêt, le peptide de liaison, et la protéine rapporteur. A priori, deux constructions sont possibles pour la protéine de fusion *Pᵢ- Π* : soit *Pᵢ* est située en N-terminal et *Π* en C-terminal, soit c'est l'inverse. Généralement, la construction avec Pi en amont est préférée. Dans un mode de réalisation alternatif, la construction inversée (*Π* en amont) est utilisée.

Dans un aspect particulier, la présente invention concerne une protéine de fusion comprenant une protéine d'intérêt ou un variant de celle-ci et une protéine rapporteur stable dans des conditions physicochimiques non standard, ainsi qu'un acide nucléique ou un vecteur codant cette protéine de fusion. Les conditions physicochimiques non standard considérées sont la haute température. Ainsi, la protéine rapporteur est thermostable. La protéine rapporteur est une protéine de résistance à un antibiotique stable dans des conditions physicochimiques non standard, et en particulier une protéine de résistance à un antibiotique thermostable. Dans un mode de réalisation tout particulièrement préféré, la protéine rapporteur thermostable est la kanamycine nucleotidyltransférase ou la protéine de résistance à la bléomycine.

Le vecteur portant la séquence codant la protéine de fusion selon la présente invention comprend tous les éléments nécessaires à son expression dans le micro-organisme hôte extrêmophile choisi. Il peut également comprendre des éléments permettant une expression dans des organimes standard. Il comprend bien entendu les éléments nécessaires à la reproduction du vecteur (par ex., origine de réplication). Le vecteur est de préférence un plasmide.

Le choix de l'organisme extrêmophile utilisé pour l'expression à l'étape b) et la sélection à l'étape c) dépend de la condition physico-chimique non standard choisie, de la facilité avec laquelle on peut transformer et faire pousser les cellules de ce micro-organisme et de la capacité de la machinerie cellulaire de ce micro-organisme à exprimer correctement la protéine *Π* et les fusions *Pᵢ-Π.*

Peuvent notamment être utilisés avec profit : des thermophiles (qui vivent à une température comprise entre 60°C et 80°C), des hyper-thermophiles (qui vivent à une température supérieure à 80°C), des psychrophiles (qui vivent à une température inférieure à 15°C), des halophiles (qui vivent avec des concentrations élevées en sel, par exemple 4M NaCl ou 3M KCl), des alkaliphiles (qui vivent à des pH supérieurs à 9), des acidophiles (qui vivent à des pH inférieurs à 3), des piezophiles (qui vivent à des pressions allant jusqu'à environ 110MPa), des metallophiles (qui vivent en présence de concentrations élevées de métaux), des radiophiles (qui vivent en présence de niveaux élevés de radiation), des microaerophiles (qui vivent malgré de très faibles teneurs en oxygène).

Par exemple, et sans que cette liste ne soit exhaustive, l'organisme extrêmophile peut être sélectionné parmi la liste suivante :
- *Thermus sp* (numéro d'accessions ATCC : 27737 ; 31674 ; 43814 ; 43815 ; 27978);
- *Thermus aquaticus* Brock and Freeze (numéro d'accessions ATCC : 25104 ; 25105 ; 31558);
- *Thermus thermophilus* Oshima and Imahori (numéro d'accessions ATCC : 27634);
- *Thermus filiformis* Hudson et al. (numéro d'accessions ATCC : 43280) ;
- *Themococus sp* (numéro d'accessions ATCC : 55659) ;
- *Alicyclobacillus acidocaldarius* (numéro d'accessions ATCC : 27009) ;
- *Sulfolobus solfataricus* Zillig et al. (numéro d'accessions ATCC : 35091 ; 35092) ; et
- *Haloferax mediterranei* (numéro d'accessions ATCC : 33500) ou des bactéries halophiles de la famille des *Halomonadaceae*

Dans un mode de réalisation préféré, le micro-organisme est thermophile et de préférence est *Thermus thermophilus* ou *Thermus aquaticus.*

Comme on vient de le voir, ces mêmes organismes peuvent également être une source intéressante de protéines *Π*.

Un problème important peut se poser, en fonction de la séquence de la protéine *P₀* de départ : celui du biais de codon. En effet, les organismes extrêmophiles ont un profil d'utilisation des codons très différent de celui des cellules non extrêmophiles, d'où est généralement issue la protéine *P₀*. En fonction de données concernant l'expression hétérologue de *P₀* dans l'organisme extrêmophile choisi, il est possible que cette différence dans les profils d'utilisation des codons empêche la bonne expression des fusions *Pᵢ- Π* à l'étape b). Il est alors nécessaire de modifier la séquence du gène codant *P₀* à l'intérieur du vecteur d'expression avant l'étape a) de manière à modifier l'utilisation des codons pour la rendre compatible avec l'expression dans l'organisme extrêmophile choisi aux étapes b) et c). A l'issue de la sélection, il peut être à nouveau nécessaire de modifier la séquence du gène codant la protéine *Pᵢ* améliorée (rendue stable) de manière à permettre son expression dans l'organisme mésophile choisi *in fine* pour la production. Par exemple, si la protéine d'origine est tirée d'un mammifère et si l'organisme utilisé pour la sélection est une bactérie thermophile, on peut modifier d'abord les codons du gène correspondant pour permettre une sélection dans un thermophile puis modifier à nouveau la séquence du « hit » obtenu à l'issue de l'évolution moléculaire pour permettre la production de la protéine améliorée dans un hôte de production classique, *Pischia pastoris* par exemple. Dans un mode de réalisation préféré, on ne modifie qu'une fois, au départ, la séquence de *P₀* de manière à ce qu'elle puisse être exprimée convenablement à la fois dans l'organisme extrêmophile utilisé pour la sélection et dans l'organisme utilisé ensuite pour la production des variants améliorés. On utilise alors un ensemble d'isocodons (codons synonymes, c'est-à-dire codant pour le même acide aminé) « consensus » qui évite de faire appel à des codons très peu utilisés dans les organismes choisis. Le problème des différences de profil d'utilisation des codons est bien connu aujourd'hui par l'homme du métier et on y est confronté, avec un degré de gravité plus ou moins important, dans pratiquement tous les cas d'expression hétérologue.

Dans un mode de réalisation particulier, les problèmes liés aux différences de profil d'utilisation des codons peuvent être résolus en réécrivant, par mutagenèse dirigée, avant l'étape a), un petit nombre (typiquement : 1-20) de codons particulièrement pénalisants pour l'expression de *P₀* (et des banques créées à partir de *P₀*) dans l'organisme extrêmophile choisi pour la sélection.

Dans un autre mode de réalisation, ces problèmes sont résolus en resynthétisant entièrement le gène codant la protéine *P₀* en concatémérisant des oligonucléotides.

Le calcul de grandeurs comme le CAI (Codon Adaptation Index ; Sharp PM et al. Nucleic Acids Res. 1987; 15:1281-95), et l'utilisation de logiciels dédiés peut guider la démarche conduisant au choix des éléments de séquence à modifier ou de la séquence à synthétiser.

De fait, pour certaines protéines *P₀,* et suivant la protéine *Π* utilisée, l'expression de la banque *Pᵢ-Π* dans un micro-organisme extrêmophile aura naturellement lieu à un niveau suffisant pour la sélection de sorte qu'aucune réédition de codons ne sera nécessaire.

La banque de variants de la protéine d'intérêt peut être préparée soit avant la fusion avec la protéine rapporteur soit après cette fusion. Dans un mode de réalisation préféré de la présente invention, la méthode comprend dans un premier temps la préparation du vecteur portant la séquence codant la protéine de fusion comprenant la protéine d'intérêt et la protéine rapporteur, puis la génération de la banque de variants de la protéine d'intérêt.

La banque de variants de la protéine d'intérêt peut être générée par une technique de mutagenèse. Dans un mode de réalisation préféré, la banque de variants de la protéine d'intérêt est créée par la technologie Massive Mutagenesis^{®}. Elle peut également être créée par une autre technologie de mutagenèse et notamment : la mutagenèse aléatoire, la mutagenèse dirigée, la mutagenèse à saturation, la mutagenèse par élongation, la mutagenèse *in vivo.*

Dans un mode de réalisation alternatif, elle peut être générée par une méthode de recombinaison génétique *in vivo* ou *in vitro.* De préférence, elle est créée à partir de la recombinaison *in vitro* circulaire (Demande de brevet FR0503364 déposée par Biométhodes) de plusieurs gènes naturels ou synthétiques codant des versions thermostables ou non de cette protéine. Cependant, elle peut également être créée à partir de la recombinaison de plusieurs gènes naturels ou synthétiques codant des versions thermostables ou non de cette protéine par une technologie de recombinaison et notamment : le DNA-Shuffling^{®}, le StEP, la recombinaison *in vivo.*

Dans un autre mode de réalisation alternatif, la banque de variants de la protéine d'intérêt est créée par clonage direct d'acides nucléiques tirés d'une source environnementale par utilisation des techniques du metagénome. L'approche metagénomique utilisée peut être de type fonctionnelle, ou de type séquence-dépendant, ou de type SIGEX.

### Mode de réalisation 1 : Mutagenèse et sélection par survie d'un thermophile à haute température.

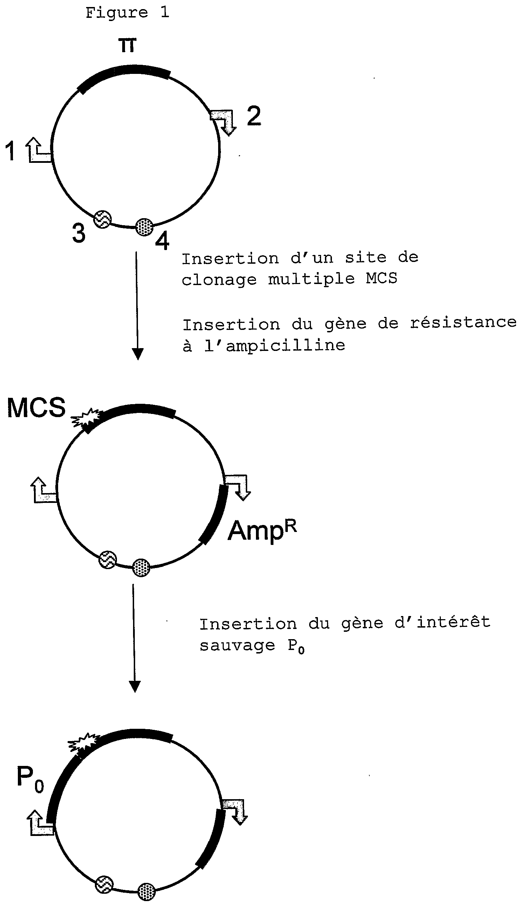

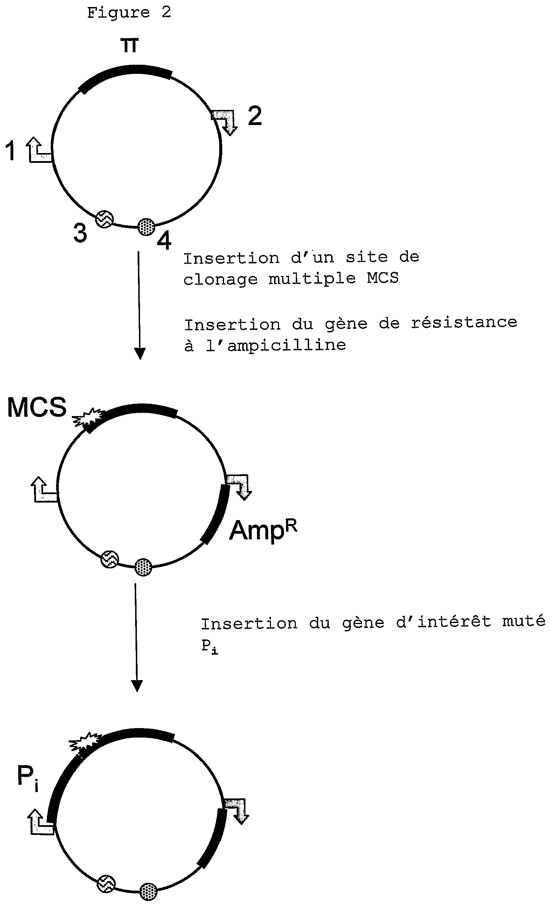

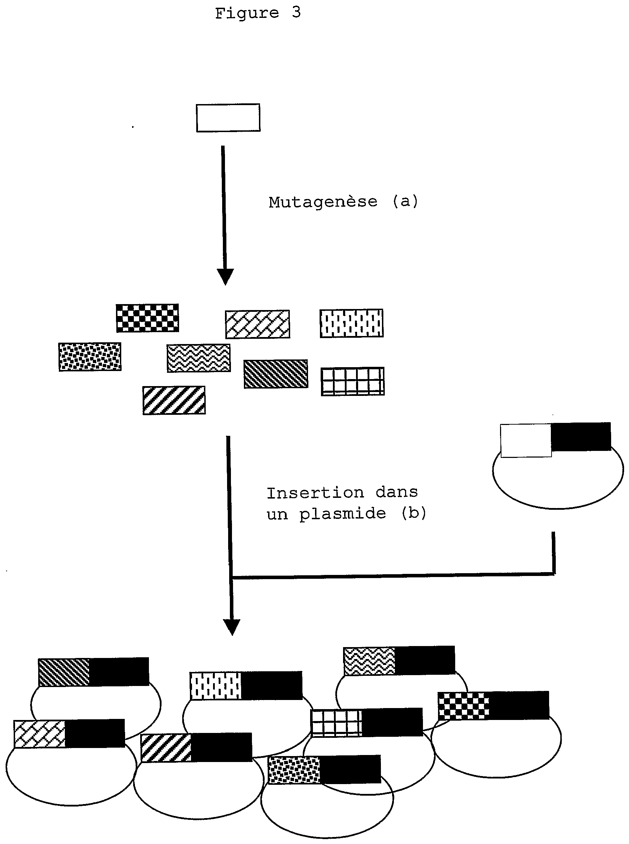

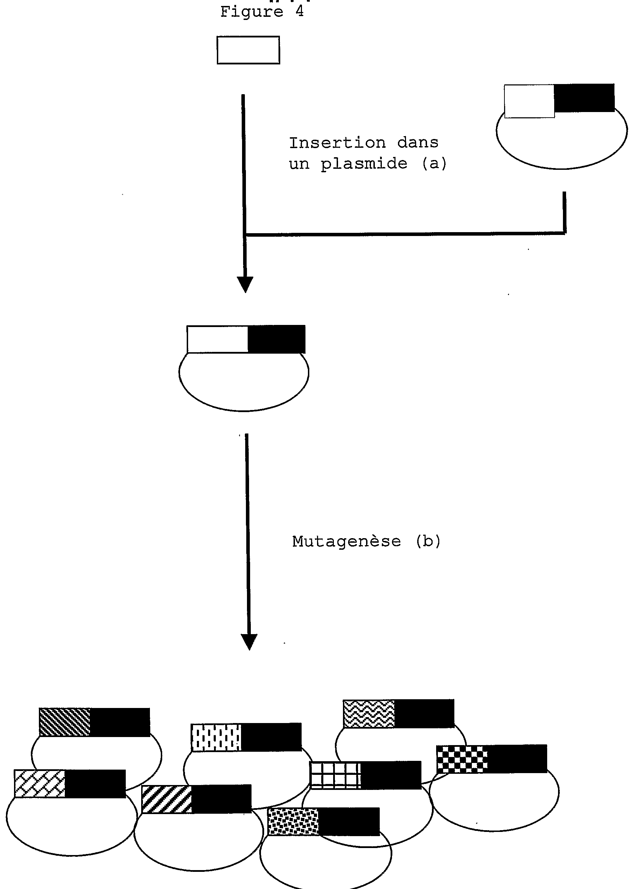

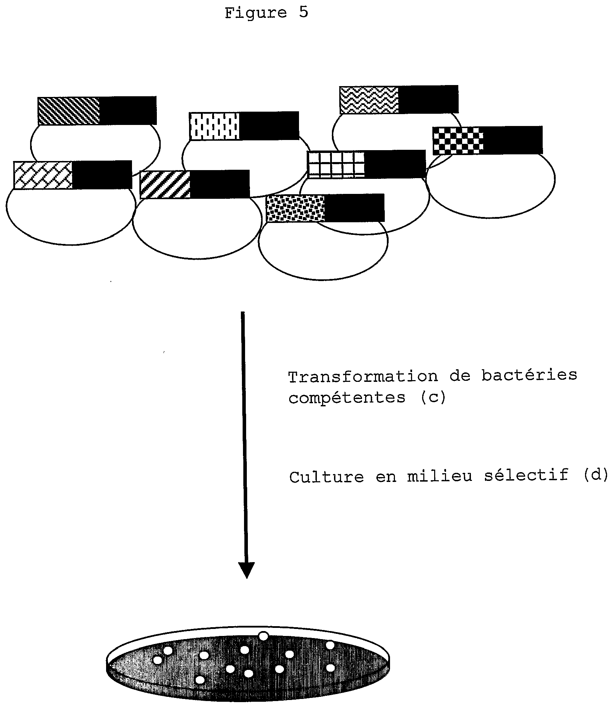

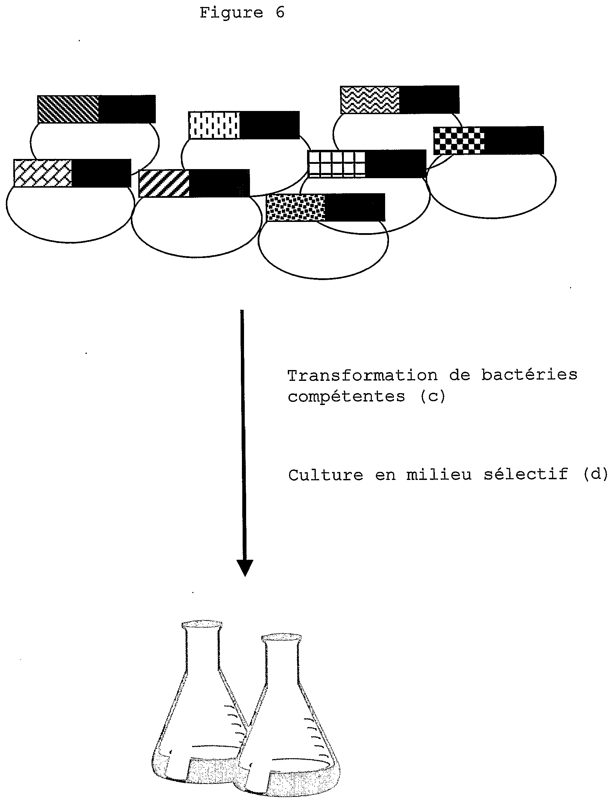

Dans un premier mode de réalisation, le procédé selon l'invention est caractérisé par l'utilisation de la mutagenèse, de la sélection par survie (figure 5) et par la succession des étapes suivantes :
a) Clonage du gène codant la protéine d'intérêt *P₀* dans un vecteur plasmidique contenant, outre toutes les séquences nécessaires à sa propre réplication, et à son expression dans un organisme thermophile, un gène codant une protéine *Π* conférant la résistance à un antibiotique à haute température. Le plasmide porte en outre une origine de réplication chez *E. Coli* et un gène de résistance à un antibiotique classique chez *E. Coli.* Le gène codant *P₀* est cloné en phase avec le gène codant *Π*, de manière à obtenir une protéine de fusion *P₀- Π,* soit en 5', soit en 3' (ce qui correspond aux deux orientations possibles de la protéine de fusion). De préférence, on insère une séquence codant un espaceur peptidique entre la protéine d'intérêt *P₀* et la protéine rapporteur thermostable *Π* afin que les deux protéines soient distantes l'une de l'autre et puissent se replier sans interférer (figures 1 et 2).
b) Génération, par utilisation d'un procédé de mutagenèse et en utilisant *E. Coli,* d'une banque de mutants *Pᵢ* (i = 1,2...N où N est compris entre 2 et 10¹⁰, et de préférence compris entre 10³ et 10¹⁰ ou entre 10³ et 10⁸). (figures 3 et 4)
c) Transformation de la banque obtenue à l'étape b) dans un micro-organisme thermophile.
d) Culture, pendant un temps nécessaire à la croissance des micro-organismes, par exemple pendant 30 minutes à 3 jours, et de préférence pendant 3 à 24 heures ou 8 à 48 heures, en milieu liquide ou solide (figure 5 ou figure 6)), à haute température (préférentiellement : 50 à 100°C) des cellules du micro-organisme thermophile, en présence de l'antibiotique thermostable.
e) Préparation de l'ADN plasmidique de la culture. Cet ADN est enrichi en gènes codant des variants thermostables de la protéine d'intérêt *P₀*.

Dans un mode de réalisation particulier, la séquence du gène codant la protéine d'intérêt *P₀* est modifiée avant l'étape a), en substituant des isocodons adaptés.

Dans un mode de réalisation particulier, d'autres substitutions d'isocodons sont également introduites après l'étape e) à l'issue de l'évolution moléculaire pour s'adapter à l'utilisation des codons dans l'organisme choisi pour la production ultérieure de la protéine améliorée. Dans un mode de réalisation préféré, la méthode de mutagenèse utilisée est la technologie Massive Mutagenesis^{®}.

Dans un autre mode de réalisation, et avec des adaptations évidentes pour l'homme du métier, la méthode de mutagenèse utilisée n'est pas Massive Mutagenesis^{®} mais la mutagenèse aléatoire, la mutagenèse dirigée ou la mutagenèse par élongation (Matsuura T et al. Nat Biotechnol. 1999; 17:58-61). Des adaptations sont en particulier nécessaires dans le cas où la méthode de mutagenèse utilisée n'opère pas sur de l'ADN circulaire mais sur de l'ADN linéaire. Il est à noter que si l'on sous-clone dans un vecteur qui comporte déjà le gène codant la protéine *P₀*, on risque d'obtenir des faux positifs (plasmides religués sans insert).

Dans un mode de réalisation particulier, et avec des adaptations évidentes pour l'homme du métier, la mutagenèse est effectuée *in vivo,* par utilisation de souches mutatrices.

Dans un mode de réalisation particulier, la banque de variants est créée à l'étape b) non pas chez *E. Coli,* mais directement dans un organisme thermophile.

Dans un mode de réalisation préféré, l'antibiotique utilisé pour la sélection dans l'organisme thermophile est la kanamycine, et le gène de résistance utilisé code la kanamycine nucleotidyltransférase thermostable.

Dans un mode de réalisation particulier, le gène codant une protéine active à haute température conférant la résistance à un antibiotique ne code pas pour la kanamycine nucleotidyltransférase, mais une autre protéine active à haute température *Π* conférant la résistance à un autre antibiotique (lui-même thermostable).

Dans un mode de réalisation particulier, et avec des adaptations évidentes pour l'homme du métier, la sélection par survie à haute température est effectuée non pas en utilisant un antibiotique, mais en faisant appel à une souche auxotrophe, en particulier une souche thermophile auxotrophe, le gène rapporteur rétablissant alors la capacité de la souche à pousser sur milieu minimum.

### Mode de réalisation 2 : Recombinaison et sélection par survie d'un thermophile à haute température.

Dans un second mode de réalisation, le procédé selon l'invention est caractérisé par l'utilisation de la recombinaison, de la sélection par survie (figure 5) et par la succession des étapes suivantes :
a) Choix de 2 à 100 (préférentiellement 2 à 10) organismes non thermophiles ou thermophiles dont les génomes codent différents variants d'une protéine d'intérêt *P₀.* Ces variants diffèrent par leur activité et par leur thermostabilité. Ce mode de réalisation permet de recombiner les gènes correspondants puis de sélectionner les nouveaux gènes ainsi créés de manière à améliorer l'activité à température élevée de la protéine d'intérêt.
b) Clonage des gènes codant les 2 à 100 (préférentiellement 2 à 10) protéines *Pᵢ*, tous dans le même vecteur plasmidique, qui contient, outre les éléments nécessaires à sa propre réplication dans un thermophile (ou un hyper-thermophile) et chez *E. Coli,* un gène de résistance à l'ampicilline permettant la sélection de clones transformants, et un gène codant une protéine rapporteur *Π* active à haute température qui confère la résistance à un antibiotique, lui-même stable à haute température. Les gènes codant *Pᵢ* sont clonés en phase avec *Π,* de manière à obtenir des protéines de fusion *Pᵢ-Π.* De préférence, les gènes codant les *Pᵢ* sont tous placés en 5' par rapport au gène codant. Alternativement, ils peuvent être placés tous en 3' (ce qui correspond aux deux orientations possibles de la protéine de fusion). De préférence, on insère en outre une séquence codant un espaceur peptidique entre la protéine d'intérêt *Pᵢ* et la protéine rapporteur *Π* afin que les deux protéines soient distantes l'une de l'autre et puissent se replier sans interférer.
c) Recombinaison, par un procédé de recombinaison génétique (ie : *in vivo* ou *in vitro,* homologue ou séquence-indépendant, aléatoire ou dirigé), des gènes *Pᵢ* qui conduit à l'obtention d'une banque.
d) Transformation de la banque obtenue à l'étape c) dans un micro-organisme thermophile.
e) Culture, pendant un temps nécessaire à la croissance des micro-organismes, par exemple pendant 30 minutes à 3 jours ou 3 à 24 heures, et de préférence 8 à 48 heures,, en milieu liquide ou solide, à haute température (préférentiellement : 50 à 100°C) des cellules de micro-organisme thermophile utilisé, en présence d'une concentration adaptée d'antibiotique thermostable.
e) Préparation de l'ADN plasmidique de la culture de l'étape e). Cet ADN est enrichi en gènes codant des variants thermostables de la protéine d'intérêt *P₀*.

Dans un mode de réalisation particulier, la séquence des gènes codant la protéine *Pᵢ* est modifiée après l'étape a) et avant l'étape b), par des mutations synonymes appropriées introduites par mutagenèse dirigée ou par resynthèse du gène pour s'adapter à l'utilisation des codons dans le micro-organisme thermophile choisi.

Dans un mode de réalisation particulier, d'autres mutations synonymes appropriées sont également introduites à l'issue de l'évolution moléculaire pour s'adapter à l'utilisation des codons dans l'organisme choisi pour la production ultérieure de la protéine améliorée.

Dans un mode de réalisation particulier, la recombinaison est effectuée en utilisant le procédé de recombinaison *in vitro* circulaire (Demande de brevet FR 0503364 déposée par Biométhodes).

Dans un mode de réalisation particulier, et avec des adaptations évidentes pour l'homme du métier, la méthode de recombinaison utilisée n'est pas la recombinaison circulaire *in vitro* mais le DNA Shuffling^{®} (Stemmer WP. Nature. 1994;370:389-91), le StEP (Zhao H. Methods Enzymol. 2004;388:42-9) ou toute autre méthode de recombinaison homologue ou séquence-indépendante adaptée. Des adaptations sont en particulier nécessaires dans le cas où la méthode de recombinaison utilisée n'opère pas sur de l'ADN circulaire mais sur de l'ADN linéaire.

Dans un mode de réalisation particulier, et avec des adaptations évidentes pour l'homme du métier, la recombinaison est effectuée *in vivo.*

Dans un mode de réalisation préféré, l'antibiotique utilisé pour la sélection dans l'organisme thermophile est la kanamycine, et le gène de résistance utilisé code la kanamycine nucleotidyltransférase thermostable.

Dans un mode de réalisation particulier, le gène codant une protéine active à haute température conférant la résistance à un antibiotique code pour une autre protéine *Π* active à haute température conférant la résistance à un autre antibiotique (lui-même thermo stable).

Dans un mode de réalisation particulier, et avec des adaptations évidentes pour l'homme du métier, la sélection par survie est effectuée non pas en utilisant un antibiotique, mais en faisant appel à une souche auxotrophe, en particulier une souche thermophile auxotrophe, le gène rapporteur rétablissant alors la capacité de la souche à pousser sur milieu minimum..

### Mode de réalisation 3 : Metagenome et sélection par survie d'un thermophile à haute

### température.

Dans un troisième mode de réalisation, le procédé selon l'invention est caractérisé par l'utilisation de techniques dite du metagenome et par la succession des étapes suivantes :
a) Choix d'une source environnementale d'ADN, par exemple, et sans évidemment que cette liste puisse être considérée comme limitative, l'eau de mer et notamment l'eau des dorsales océaniques, le sol, et notamment la rhizosphère, l'air, la glace de l'antarctique,
b) A partir de l'ADN extrait de cette source environnementale, clonage direct par la technique du metagénome (Streit WR et al. Curr Opin Biotechnol. 2004;15:285-90 ; Daniels R Curr Opin Biotechnol. 2004; 15:199-204), selon la technique du criblage séquence-dépendant, du criblage fonctionnel ou encore du SIGEX (« substrate-induced gene-expression screening » ; Yun J et al. Microb Cell Fact. 2005; 4:8) d'un nombre indéterminé de variants *Pᵢ* d'une protéine d'intérêt *P₀* dans un même vecteur plasmidique, contenant, outre tous les éléments nécessaires à sa propre réplication et à l'expression (dans un thermophile ou un hyper-thermophile et aussi dans un mésophile), d'un gène codant une protéine rapporteur thermostable *Π* qui confère la résistance à un antibiotique (lui-même thermostable). Les gènes codant *Pᵢ* sont clonés en phase avec *Π*, de manière à obtenir une protéine de fusion *Pᵢ-Π.* Une banque est obtenue. Soit les gènes codant les *Pᵢ* sont tous placés en 5' par rapport au gène codant, soit ils sont placés tous en 3' (ce qui correspond aux deux orientations possibles de la protéine de fusion). De préférence, on insère en outre une séquence codant un espaceur peptidique entre la protéine d'intérêt *Pᵢ* et la protéine thermostable *Π* afin que les deux protéines soient distantes l'une de l'autre et puissent se replier sans interférer.
c) Transformation de la banque obtenue à l'étape b) dans le micro-organisme thermophile de sélection.
d) Culture, un temps nécessaire à la croissance des micro-organismes, par exemple pendant 30 minutes à 3 jours ou 3 à 24 heures, et de préférence 8 à 48 heures" en milieu liquide ou solide, à haute température (préférentiellement : 50 à 100°C) des cellules du micro-organisme thermophile de sélection, en présence d'une concentration adaptée d'antibiotique thermostable.
e) Préparation de l'ADN plasmidique de la culture. Par rapport à la banque initiale, cet ADN est enrichi en gènes codant des protéines *Pᵢ* thermostables.

Dans un mode de réalisation particulier, des mutations synonymes appropriées sont introduites à l'issue de l'évolution moléculaire pour s'adapter à l'utilisation des codons dans l'organisme choisi pour la production ultérieure de la protéine améliorée.

Dans un mode de réalisation préféré, l'antibiotique utilisé pour la sélection dans l'organisme thermophile est la kanamycine, et le gène de résistance utilisé code la kanamycine nucleotidyltransférase thermostable.

Dans un mode de réalisation particulier, le gène codant une protéine active à haute température conférant la résistance à un antibiotique code pour une autre protéine *Π* active à haute température conférant la résistance à un autre antibiotique (lui-même thermo stable).

Dans un mode de réalisation particulier, et avec des adaptations évidentes pour l'homme du métier, la sélection par survie est effectuée non pas en utilisant un antibiotique, mais en faisant appel à une souche auxotrophe, en particulier une souche thermophile auxotrophe, le gène rapporteur rétablissant alors la capacité de la souche à pousser sur milieu minimum.

### Mode de réalisation 4 : Criblage par FACS

Dans un quatrième mode de réalisation, le procédé selon l'invention est caractérisé par l'utilisation d'une protéine *Π* fluorescente qui est stable dans des conditions physico-chimiques non standard. La protéine peut être directement active dans ces conditions physico-chimiques non standard ; alternativement, elle peut être produite dans ces conditions physico-chimiques non standard sans être active, puis devenir active lorsqu'on la replace dans des conditions standard.

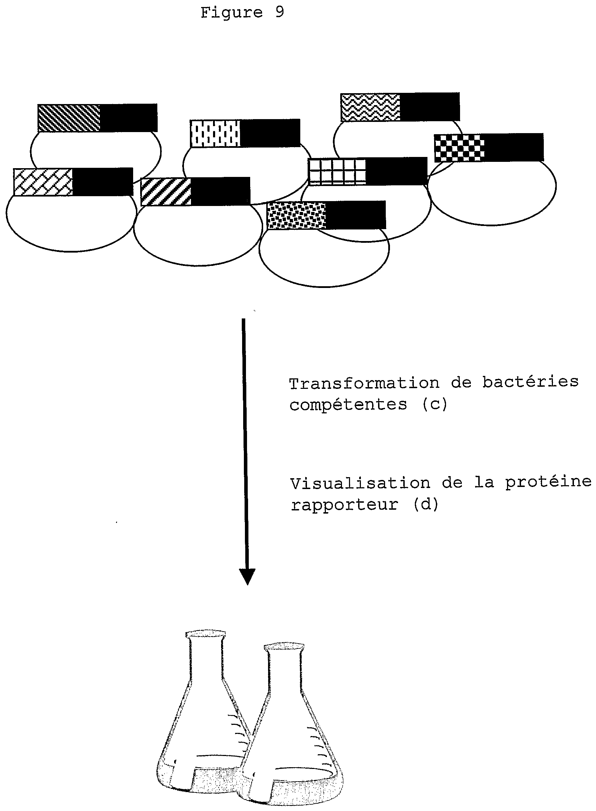

Le tri a lieu en utilisant la cytométrie de flux et un FACS (fluorescence activated cell sorter ; figure 9). Ce mode de réalisation relève plus du criblage haut-débit que de la sélection, puisque les cellules sont triées une par une. Le débit est cependant relativement similaire au cas de la sélection sur un critère de survie : typiquement 10² à 10⁴ cellules triées par seconde au FACS, soit la possibilité de cribler des banques de l'ordre de 10⁷ variants en une heure).

Dans ce quatrième mode de réalisation, le procédé selon l'invention est caractérisé par la succession des étapes suivantes :
a) Construction d'une banque de plasmides contenant tous les éléments nécessaires à leur réplication et à l'expression (chez un extrêmophile ainsi que chez le mésophile éventuellement utilisé pour la génération de diversité au départ ou la production de la protéine améliorée à la fin), et contenant une séquence codant une protéine de fusion *Pᵢ*-*Π*, où *Pᵢ* est un variant de la protéine d'intérêt (ces variants étant obtenus par une technique de mutagenèse, de recombinaison ou encore par les techniques du metagenome) et où *Π* est une protéine fluorescente ou luminescente (par exemple, la GFP ou un variant de la GFP). Préférentiellement la protéine de fusion intègre un espaceur peptidique adapté. Il est fondamental que la protéine *Π* utilisée soit non seulement stable à haute température mais puisse être produite par expression à haute température dans un organisme thermophile (figure 1).
b) Transformation d'un organisme extrêmophile par une banque de plasmides (figures 3 et 4).
c) Expression dans des conditions physico-chimiques non standard (par exemple, suivant l'extrêmophile choisi, à une température de 75°C, ou à une concentration de 3M en NaCl, ou à pH 2) de la banque.
d) Tri, à basse température (préférentiellement, de 5°C à 45°C) et par utilisation d'un FACS, ou d'un FACS miniaturisé (µFACS), ou encore de nombreux µFACS utilisés sur un mode parallèle, des cellules de l'organisme extrêmophile. On récupère la fraction des cellules qui émettent, lorsqu'elles sont excitées à une longueur d'onde appropriée, une fluorescence mesurée au-dessus d'un seuil déterminé empiriquement (par exemple, on récupère les 0.1% de cellules qui fluorescent le plus).
e) Préparation de l'ADN plasmidique de ces cellules. Par rapport à la banque initiale, cet ADN est enrichi en gènes codant des protéines thermostables.

Dans un mode de réalisation particulier, on réalise, par mutagenèse dirigée ou par synthèse de gènes, les substitutions d'isocodons nécessaires à une bonne expression hétérologue.

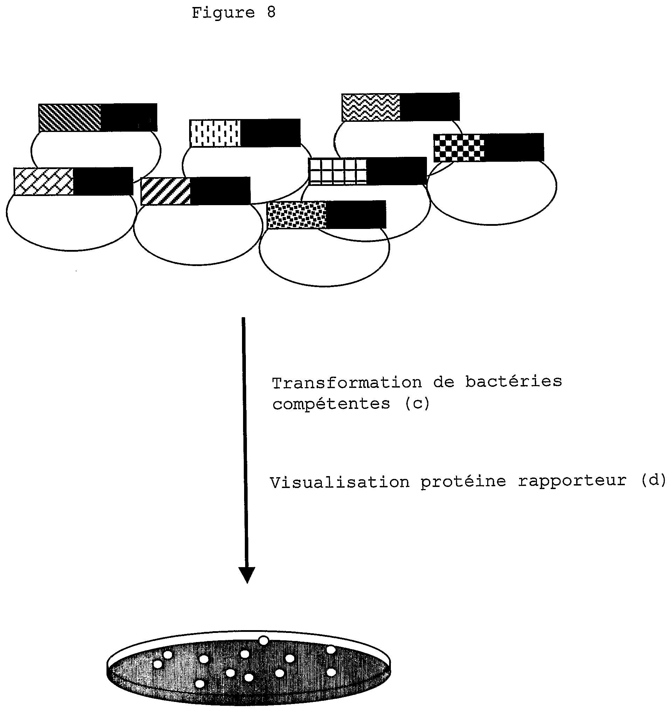

Dans un mode de réalisation particulier, la sélection n'a pas lieu par FACS, mais par lecture de la fluorescence de colonies cultivées sur support solide puis prélèvement et repiquage des colonies présentant une fluorescence élevée (figure 8).

### Exemples.

### Exemple 1 : Exemple de plasmide.

Un plasmide pouvant être utilisé dans le procédé selon l'invention comporte, dans un ordre approprié, et de façon à ce qu'une phase de lecture unique soit respectée, les éléments suivants :
a) une origine de réplication dans un extrêmophile (utilisé pour la sélection), et éventuellement une autre origine de réplication dans un mésophile (éventuellement utilisé pour l'obtention de diversité génétique ou pour la production de la protéine améliorée).
b) un ou plusieurs promoteurs, éventuellement un ou plusieurs promoteurs qui permet(ent) une expression conditionnelle;
c) un gène de résistance à l'ampicilline ou à un autre antibiotique classique, sous contrôle d'un promoteur adapté, qui permet la sélection de transformants chez *E. coli* ou tout autre système classique adapté (par exemple, si on utilise des levures, ce gène est de préférence un marqueur d'auxotrophie);
d) un gène codant une protéine rapporteur stable dans des conditions physico-chimiques non standard et éventuellement active dans ces conditions;
e) facultativement, une portion de séquence codant un peptide de liaison d'une dizaine d'acides aminés;
f) un gène codant la protéine d'intérêt ou un variant de celle-ci.

### Exemple 2 : Amélioration de la thermostabilité d'une phytase.

Augmenter la thermostabilité de phytases bactériennes permet de répondre à un enjeu économique et environnemental majeur dans le domaine de l'alimentation animale. La phytase d'*Escherichia coli* (myo-inositol hexakisphosphate phosphohydrolase, EC 3.1.3.8), catalyse le clivage de l'acide phytique, la forme majeure de stockage du phosphore dans les graines de plantes, avec libération d'un phosphomonoester. Le phosphore présent dans l'acide phytique n'est pratiquement pas utilisable par les animaux monogastriques comme les porcs et la volaille. Aussi, supplémenter l'alimentation de ces animaux avec une phytase augmente la valeur nutritionnelle de l'alimentation fournie. En réduisant le besoin de suppléments en phosphate inorganique dans l'alimentation animale, les phytases aident aussi à préserver l'environnement. Les procédés impliqués dans la fabrication des granulés ou autres aliments pour animaux font très généralement intervenir des étapes à haute température (typiquement : une phase courte de 1 seconde à trois minutes, à 80°C ou plus). Il est donc nécessaire de disposer d'une phytase qui est stable à ces hautes températures, tout en étant active à la température du corps si on veut pouvoir l'incorporer dans l'alimentation animale.
a) Le gène PhyA codant la phytase d'*Escherichia coli* est cloné en phase avec le gène codant la kanamycine nucléotidyltransférase thermostable. Une séquence codant un peptide de liaison d'une dizaine d'acides aminés est insérée entre les deux gènes. Le vecteur contient en outre un gène de résistance à l'ampicilline (non thermostable) sous le contrôle d'un promoteur bactérien, et tous les éléments nécessaires à la réplication dans *T. Thermophilus* et *E. Coli.*
b) En utilisant des oligonucléotides adaptés, on applique la technique de mutagenèse site-dirigé haut-débit Massive Mutagenesis^{®} au plasmide contenant la construction de l'étape a) pour contruire une banque de variants du gène PhyA.
c) On transforme un organisme thermophile *(Thermus thermophilus),* avec le plasmide contenant la banque de variants du gène PhyA obtenue à l'étape b).
d) On cultive, pendant plusieurs heures, à une température de 70°C, l'organisme thermophile en présence d'une concentration adaptée de kanamycine. Après 48 heures de pousse en milieu liquide, on observe une croissance bactérienne significativement plus importante que dans le lot transformé par le plasmide non muté, utilisé ici comme témoin.
e) On prépare l'ADN plasmidique des cellules cultivées à l'étape d).
f) On transforme *E. Coli* avec cet ADN plasmidique et on étale sur une boîte de pétri contenant de l'ampicilline.
g) Après avoir laissé pousser une nuit, on repique dans une dizaine de boîtes à 96 puits des colonies individuelles prélevées sur la boîte de pétri de l'étape f).
h) On se place dans des conditions permettant l'expression de la phytase dans *E. Coli.* Il aura été préalablement confirmé que l'activité de ladite phytase n'est pas altérée par la présence du gène rapporteur fusionné.
i) On lyse les cellules bactériennes.
j) On utilise un test adapté pour cribler rapidement les clones exprimant une phytase qui, en plus d'avoir acquis de la thermostabilité, est active à basse température (37°C).

Dans un mode de réalisation particulier, l'activité phytase est mesurée directement dans le surnageant de culture. Pour cela, 20µL de surnageant de culture sont incubés avec 200µL d'une solution de phytate de sodium (10g/L dans du tampon acétate de sodium 250mM - CaCl2 1mM - pH5,5) à 37°C pendant des temps variables (de 15min à 18h). A t=0 et à la fin de la réaction, 50µL de la réaction sont prélevés et mélangés à 50µL de TCA 20% pour arrêter la réaction. L'activité phytase est estimée par le dosage des phosphates libérés dans le milieu réactionnel. Ce dosage s'effectue par ajout de 100µL d'un mélange 4:1 de molybdate d'ammonium 12mM et de sulfate de fer 380mM. Après 30min d'incubation à 20°C (température ambiante), la DO est mesurée à 620nm. Le facteur d'augmentation du signal est calculé par le rapport (DO finale) / (DO initiale).

### Exemple 3 : Amélioration de la résistance aux pH extrêmes d'une cellulase.

Les cellulases peuvent être utilisées dans la conversion de biomasse mais aussi dans divers procédés industriels, dont l'agroalimentaire, le textile, la lessive, le papier. En vue de l'utiliser dans plusieurs de ces procédés, on souhaiterait disposer d'une cellulase possédant une activité spécifique élevée à des pH neutres ou alcalins (pH 6 à pH 11). Des auteurs ont récemment décrit l'obtention, par évolution dirigée, d'un variant d'une cellulase, l'endoglucanase III (EG III) issu de *Trichoderma reesei* (Wang T et al. Biomol Eng. 2005; 22: 89-94) ayant un optimum de pH (5.4) augmenté de 0.6 unités par rapport à l'enzyme sauvage. On cherche à aller plus loin dans cette amélioration. Dans le cas d'une cellobiohydrolase de la famille 7 des glycosyl hydrolases (Cel7), des auteurs ont mis en évidence une corrélation très claire entre thermostabilité et activité à pH alcalins (Boer H et al. Eur. J. Biochem. 2003; 270: 841-848).
a) Le gène Cel7 codant la cellulase Cel7 de *Trichoderma reesei* est cloné en phase avec le gène codant la kanamycine nucléotidyltransférase thermostable. Une séquence codant un peptide de liaison d'une dizaine d'acides aminés est insérée entre les deux gènes.
b) En utilisant des oligonucléotides adaptés, on applique la technique de mutagenèse site-dirigé haut-débit Massive Mutagenesis^{®} au plasmide contenant la construction de l'étape a) pour contruire une banque de variants du gène Cel7.
c) On transforme un organisme thermophile *(Thermus thermophilus)* avec le plasmide contenant la banque obtenue à l'étape b).
d) On cultive, pendant plusieurs heures, à une température de 70°C, l'organisme thermophile en présence d'une concentration adaptée de kanamycine.
e) On prépare l'ADN plasmidique des cellules cultivées à l'étape d).
f) On coupe les plasmides obtenus autour du gène d'intérêt et sous-clone l'insert dans un plasmide permettant son expression par *E. coli.* Ce plasmide comporte un gène de résistance à la tétracycline. Ce sous-clonage permet de s'affranchir de la présence du gène rapporteur fusionné, susceptible de gêner l'activité de la protéine et donc d'empêcher la caractérisation fonctionnelle des mutants plus stables obtenus lors les étapes précédentes.
g) On transforme *E. Coli* avec cet ADN plasmidique et on étale sur une boîte de pétri contenant de la tétracycline.
h) Après avoir laissé pousser une nuit, on repique dans une dizaine de boîtes à 96 puits des colonies individuelles prélevées sur la boîte de pétri de l'étape f)
i) On se place dans des conditions permettant l'expression de la cellulase dans *E. Coli.*
j) On lyse les cellules bactériennes.
k) On utilise un test adapté pour cribler rapidement les cellulases ayant une activité spécifique importante à un pH alcalin, afin d'isoler, parmi ces mutants associés à un gain de stabilité, ceux qui ont conservé un niveau d'activité significatif.

### Exemple 4 : Amélioration de la stabilité in vivo d'une cytokine.

a) On part du gène IL7, qui code une cytokine présentant un potentiel en tant que protéine thérapeutique pour l'homme et dont on voudrait améliorer la stabilité *in vivo* dans l'organisme humain. La démarche suivie consiste à chercher des mutants plus thermostables en utilisant le procédé de l'invention, et en espérant qu'une partie significative de ces mutants thermostables seront également plus résistants à la protéolyse, et auront également une demi-vie augmentée en conditions plus douces, comme précédemment décrit dans la littérature. Ce gène est totalement synthétisé (par la méthode de la PCR chevauchante en assemblant de longs oligonucléotides d'une centaine de base synthétisés chimiquement) en modifiant la séquence du gène humain de manière à obtenir des substitutions d'isocodons qui permettent sa bonne expression chez *Thermus thermophilus* et chez *E. coli.* Ce gène resynthétisé est cloné en phase avec le gène codant la kanamycine nucléotidyltransférase thermostable. Une séquence codant un peptide de liaison d'une dizaine d'acides aminés est insérée entre les deux gènes.
b) En utilisant des oligonucléotides adaptés, on applique la technique de mutagenèse site-dirigé haut-débit Massive Mutagenesis^{®} au plasmide contenant la construction de l'étape a) pour contruire une banque de variants du gène IL7.
c) On transforme un organisme thermophile (*Thermus thermophilus),* avec le plasmide contenant la banque obtenue à l'étape b).
d) On cultive, pendant plusieurs heures, à une température de 70°C, l'organisme thermophile en présence d'une concentration adaptée de kanamycine.
e) On prépare l'ADN plasmidique des cellules cultivées à l'étape d).
f) On transforme *E. coli* avec cet ADN plasmidique et on étale sur une boîte de pétri contenant de l'ampicilline.
g) Après avoir laissé pousser une nuit, on repique dans une dizaine de boîtes à 96 puits des colonies individuelles prélevées sur la boîte de pétri de l'étape f)
h) On se place dans des conditions permettant l'expression de la cytokine dans *E. Coli.*
i) On lyse les cellules bactériennes.
j) On utilise un test adapté pour cribler rapidement les clones exprimant une cytokine ayant une activité spécifique importante à 37 °C, après s'être assuré que la présence du gène rapporteur fusionné n'altérait pas l'activité del'IL7.
k) Un criblage secondaire, sur un nombre réduit de clones sélectionnés à l'étape i) est effectué sur le critère de la demi-vie plasmatique de la cytokine.

### Exemple 5 : Création d'une enzyme halotolérante en utilisant un halophile (Exemple non-illustratif de l'invention).

a) On part d'une banque de variants d'un gène codant une enzyme issue d'un organisme mésophile, qui est active en milieu aqueux mais très peu ou pas active à de très hautes concentrations d'un substrat salin, conditions dans lesquelles on voudrait l'utiliser (ce qui représente une problématique classique dans le domaine de la biocatalyse). Une façon d'y parvenir est d'améliorer la tolérance au sel de cette enzyme, en utilisant une bactérie halophile de la famille des *Halomonadacea (*Halomonas, Chromohalobacter, Zymobacter), qui peuvent être manipulée génétiquement (Vargas, C et al. J. Methods Mol Biol. 2004;267:183-208). Cette banque de gène est clonée en phase avec un plasmide qui permet de l'exprimer sous forme d'une protéine de fusion avec une protéine rapporteur conférant la résistance à un antibiotique, cette protéine rapporteur étant stable et active dans les conditions de vie de l'halophile. Le plasmide comporte en outre une origine de réplication chez *E. Coli* et un gène de résistance à l'ampicilline chez *E. Coli.*
b) On transforme l'organisme halophile par la construction de l'étape a) et on met en culture, dans les conditions de vie de l'halophile (donc : en présence d'une quantité importante voire saturante d'un ou plusieurs sels), l'organisme halophile, en présence de l'antibiotique et dans des conditions permettant l'expression de la banque de protéines de fusion. On peut utiliser un milieu solide ou un milieu liquide.
c) On prépare l'ADN plasmidique des clones ayant poussé.
d) On transforme *E. coli* avec cet ADN plasmidique et on étale sur une boîte de pétri contenant de l'ampicilline.
e) Après avoir laissé pousser une nuit, on repique dans une dizaine de boîtes à 96 puits des colonies individuelles prélevées sur la boîte de pétri de l'étape d)
f) On se place dans des conditions permettant l'expression de l'enzyme dans *E. Coli.*
g) On lyse les cellules bactériennes.
h) On utilise un test adapté pour cribler rapidement les clones exprimant une enzyme active en utilisant le substrat d'intérêt, après s'être assuré que la présence du gène rapporteur fusionné n'altérait pas l'activité de l'enzyme d'intérêt.

### Exemple 6 : Amélioration de la stabilité d'une cytokine : obtention de mutants thermostables de l'Interferon gamma.

En utilisant un mutant thermostable de la kanamycine nucleotidyltransférase (KNTase) comme marqueur de sélection (Liao HH Enzyme Microb. Technol. 1993; 15:286-92; Sakon J et al. Biochemistry. 1993; 32: 11977-84) et la souche HB27 de *Thermus thermophilus* (dont la température optimale de croissance se situe autour de 70°C) comme hôte de sélection, des mutants thermostables de l'interféron gamma (IFNγ) ont été rapidement identifiés.

Un vecteur navette *E. Coli*/ *T. Thermophilus* a d'abord été généré. Le gène de l'IFNγ humain a été cloné dans ce vecteur en phase avec le marqueur de sélection. Une banque de mutants a été réalisée en utilisant la technologie Massive Mutagenesis®. Des mutants ont été identifiés sur le critère de la résistance à haute concentration de kanamycine. Les mutations ont été identifiées par séquençage. Les mutations ont ensuite été introduites dans un vecteur d'expression mammifère permettant la production d'IFNγ, et l'amélioration de thermostabilité a été validée dans un modèle de culture cellulaire.

### Construction d'un vecteur navette E.coli / T.thermophilus

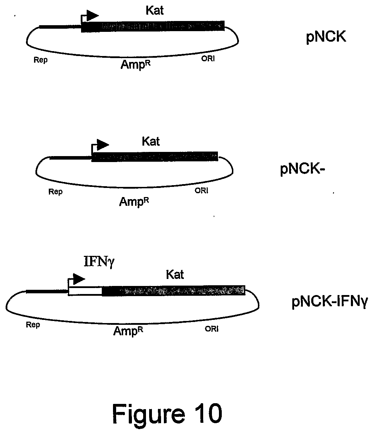

Le vecteur qui a été utilisé, appelé pNCK, comportait les origines de réplication de E. coli et de T. Thermophilus, un gène de résistance à l'ampicilline qui permet la sélection de transformants dans E. Coli ainsi qu'un gène codant la KNTase thermostable sous contrôle d'un promoteur actif à la fois chez E. Coli et T. Thermophilus (le promoteur ps1pA) (Figure 10). La séquence nucléotidique de l'IFNγ (SEQ ID No 5 codant pour la forme mature de 146 acides aminés SEQ ID No 6) a été clonée entre les sites NcoI et NotI du vecteur pNCK en N-terminal de la KNTase (pNCK-IFNγ). L'IFNγ et la KNTase en fusion ont été séparés par une séquence codant un peptide de liaison (« linker ») qui présentait la séquence peptidique AAAGSSGSI (SEQ ID No 8) et était codée par la séquence nucléique GCG-GCC-GCA-GGA-AGC-TCT-GGT-TCC-ATC (SEQ ID No 7).

### Mise en place du système de sélection

Deux mutants thermostables décrits dans la bibliographie ont été également construits et utilisés à titre de contrôles positifs. Ils codent pour :
- la protéine IFNγ E30C/S92C (Waschutza et al., 1996)
- la protéine IFNγ Δ10 (extrémité C-terminale de la protéine délétée des 10 derniers acides aminés (Slodowski et al., 1991).
   Un témoin négatif a également été ajouté; ce mutant comporte un codon stop 30 pb avant la fin de la séquence de l'IFNγ.

Ces différentes constructions ont ensuite été transformées chez *T. Thermophilus.* Ont également été transformés en parallèle :
- le plasmide pNCK vide (témoin positif 1 = possède la KNTase sous promoteur pslpA et le linker en N-terminal)
- le plasmide pNCK- (témoin positif 2 = possède la KNTase sous promoteur pslpA mais sans peptide linker)

La souche de *T. Thermophilus* a été transformée à 70°C (par compétence naturelle) avec des quantités égales de chaque plasmide. Après une période d'incubation de 4 heures, 10 μl d'une dilution 10⁻³ du mélange de transformation ont été «spottés» sur boites contenant différentes concentrations de kanamycine et incubés à 60°C et 70°C.

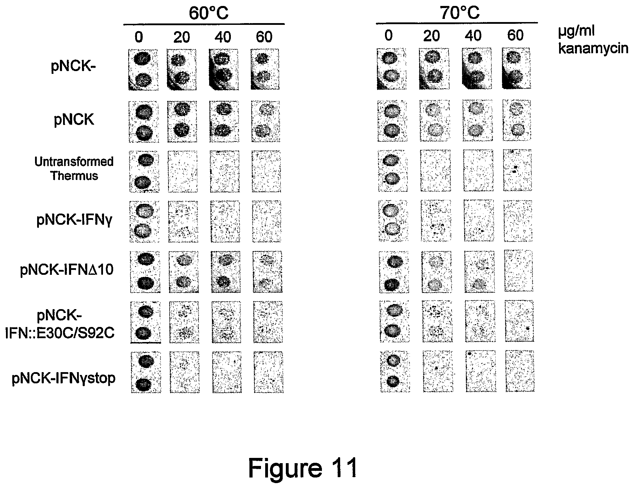

Les résultats, présentés sur la figure 11, correspondent à la pousse des différents transformants après 48 heures d'incubation aux deux températures. On remarque que :
- la souche non transformée ne poussait qu'en absence de kanamycine ;
- les témoins positifs pNCK et pNCK- montraient un niveau de résistance très élevé ;
- la transformation par le plasmide pNCK-IFNγ a confèré une certaine résistance à la souche transformée ;
- les différents contrôles se sont comportés comme espéré:
- le mutant présentant un codon stop (contrôle négatif) ne présentait pratiquement pas de signal ;
- les mutants décrits comme thermostables ont été plus résistants à la kanamycine que pNCK-IFNγ à haute température.

Il est donc possible de discriminer des variants plus ou moins thermostables de l'IFNγ, grâce à la fusion de celle-ci avec la KNTase thermostable et la transformation dans *T*. *Thermophilus.* De plus, la gamme de résistance qui a pu être déterminée permet de dégager des conditions pour sélectionner des variants plus stables contenus dans une banque de variants de pNCK-IFNγ (20 à 40 μg/ml à 70°C).

### Génération d'une banque de mutants

Une banque de variants de l'IFNγ cloné dans le vecteur pNCK a été générée en utilisant la technologie Massive Mutagenesis®. Une diversité totale a été introduite sur toutes les positions situées entre 21 et 166. La banque de mutants contient potentiellement tous les 2800 mutants simples, une partie significative des 4 millions de mutants doubles, ainsi que des mutants ayant trois mutations ou plus.

### Sélection de mutants thermostables de l'IFNγ

La banque a ensuite été transformée par compétence naturelle à haute température (70°C) dans *T. Thermophilus* et sélectionnée à 20 ou 40 μg/ml de kanamycine.

Les différents mutants isolés par le moyen de cette sélection primaire ont ensuite été retransformés dans *T. Thermophilus* et leur niveau de résistance a été comparé à celui de la construction sauvage. 20 mutants, conférant à la souche une résistance plus ou moins importante mais toujours supérieure au sauvage ont ainsi été confirmés. Pour ces 20 mutants, un facteur de thermostabilité (TF) a été calculé en divisant le nombre de clones obtenus en conditions sélectives (20 ou 40 μg/ml de kanamycine et 70°C) par le nombre de clones obtenus en conditions non sélectives (20μg/ml et 60°C), ceci pour s'affranchir d'éventuels biais dus à l'efficacité de transformation de chaque clone. On a observé que les mutants ayant été sélectionnés à faible stringeance sont associés à un facteur de thermostabilité moins important que les mutants sélectionnés à forte stringeance.

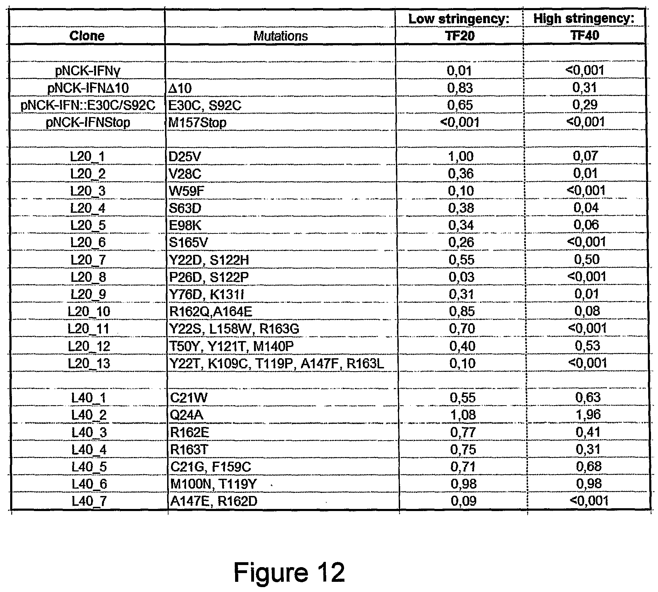

35 mutations ont été identifiées par séquençage des 20 clones: C21G, C21W, Y22D, Y22S, Y22T, Q24A, D25V, P26D, V28C, T50Y, W59F, S63R, Y76D, E98K, M100N, K109C, T119P, T119Y, Y121T, S122H, S122P, K131I, M140P, A147E, A147F, L158W, F159C, R162D, R162E, R162Q, R163G, R163T, R163L, A164E, S165V, La séquence des mutants est répertoriée dans la figure 12, avec leurs facteurs de thermostabilité.

### Validation de la stabilité des mutants de l'IFNγ en cellules de mammifère

Certaines des 35 mutations simples précédemment identifiées ont été introduites indépendamment dans le vecteur pORF/IFNγ (Invivogen), permettant d'exprimer transitoirement l'IFNγ en cellules mammifères grâce à un promoteur hybride (EF-1α-HLTV) et un signal de polyadénylation fort du SV40.

La transfection des cellules COS7 a été réalisée en plaque 24 puits avec un ensemencement de 30 000 à 60 000 cellules par puits lorsque les cellules atteignent 70-80% confluence. La transfection a été réalisée avec environ 50ng d'ADN et du Jet PEI (Polyplus transfection) en utilisant un ratio Jet PEI/ADN de 5 en laissant 30 minutes à température ambiante. Après 24h de transfection, le milieu (500μL IMDM + SVF + antibiotiques) a été changé. Les surnageants contenant l'IFNγ (avec un niveau d'expression de l'ordre de 0,5 à 1μg/ml) ont été récupérés 24 heures après la transfection. Ils ont été aliquotés et conservés à -20°C avant le dosage de l'activité de l'IFNγ.

Il a été déjà été décrit que l'IFNγ active spécifiquement les récepteurs à l'IFNγ présents sur les cellules HeLa. La stimulation de la voie des Jak/Stat1 des cellules HeLa par l'IFNγ s'effectue en ayant, en particulier, pour conséquence l'activation de la transcription des gènes sous le contrôle de promoteur possédant des séquences GAS pour « Gamma Activated Site ». Il est alors possible de mesurer et de comparer les activités des variants de l'IFNγ en transfectant dans les cellules HeLa un système de gène rapporteur dans lequel la luciférase (firefly luciferase) est en aval d'un promoteur possédant plusieurs sites GAS (plasmide pGAS/Luciférase de Stratagene).

Les transfections de cellules HeLa à 50-80 % de confluence ont été réalisées en plaque 96 puits selon le protocole du fournisseur : 20 000 cellules par puits ont été transfectées avec environ 150ng d'ADN pGAS/Luciférase et du jet PEI dans un rapport Jet PEI/ADN de 5. Le tout a été vortexé 30s et laissé à température ambiante 30 min. Puis, 20μL du mélange ADN/Jet PEI ont été répartis dans chaque puits de la plaque et les cellules ainsi transfectées sont cultivées pendant 24 heures à 37°C et en 5% CO2.

Tous les réactifs de culture cellulaire ont été fournis par Invitrogen. Les cellules HeLa et les cellules COS-7 ont été cultivées dans des conditions standard (37°C en atmosphère humide contenant 5% CO2) en utilisant respectivement les milieux Dulbecco's Modified Eagle's Medium (D-MEM) et Iscove's Modified Dulbecco's Medium (IMDM). Tous ces milieux de culture contiennent un analogue de la L-glutamine (le glutamax) et sont supplémentés en sérum de veau foetal décomplémenté (10% final) et en antibiotiques à raison de 100 unités/ml de pénicilline et 0.1 mg/ml de streptomycine. Le vecteur pSV-betagal ™ (Promega), qui exprime la béta galactosidase sous le contrôle du promoteur précoce SV40, a été utilisé pour normaliser les efficacités de toutes les transfections réalisées.

On a ensuite fait agir les surnageants de cellules COS7 transfectées par les mutants de l'IFNγ sur des cellules HeLa transfectées : Les surnageants de cellules COS7 contenant l'IFNγ ont été dilués au 1/100ème. 10μL de ces dilutions de surnageants de cellules COS7 contenant l'IFNγ ont été ajoutés sur les cellules HeLa transfectées par du pGAS/Luciférase. Après 16 heures à 37°C 5% CO2 pendant lesquelles l'expression cytoplasmique de la *firefly* luciférase s'est effectuée, les culots de cellules ont été récupérés et congelés. 50μL de Glo lysis buffer™ (Promega), ont été ajoutés pour lyser les cellules. La lyse s'est effectuée pendant 10 min sous agitation à température ambiante de façon à libérer la luciférase produite en réponse à la stimulation spécifique de l'IFNγ. Le test de l'activité luciférase à proprement dit a été initialisé par l'ajout du réactif Bright Glo™ (Promega) et la quantité de luciférase accumulée a ensuite été comptée avec un luminomètre (FLX 800, Bio-Tek Instrument).

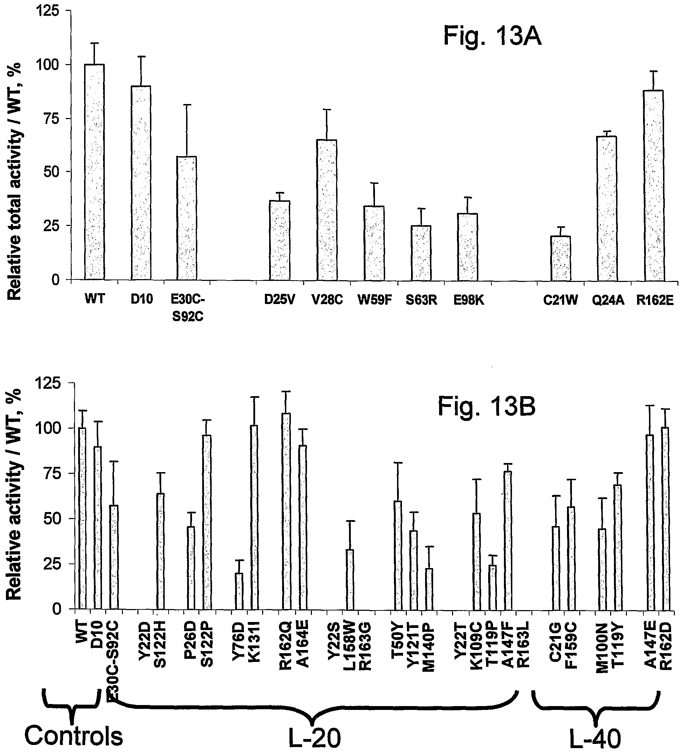

Le calcul de l'activité totale (par rapport à la protéine sauvage) de chaque variant est une moyenne réalisée sur la base de résultats obtenus sur 5 manipulations différentes (duplicates au minimum) et sur des surnageants de culture provenant, au minimum, de deux transfections indépendantes. Les barres d'erreur présentées sont calculées par la formule de l'erreur standard sur la moyenne (s.e.m). L'une des façons de présenter les résultats d'activité totale est de rapporter l'activité de base de chaque variant en pourcentage de l'activité de base de l'IFNγ non muté exprimé dans les mêmes conditions pour chaque transfection (figure 13). Une partie des mutants ont gardés une activité égale ou proche de celle de l'IFNγ de type sauvage.

### Mesure du niveau d'activité et du niveau de thermostabilité

Les surnageants de culture de cellules COS7 ont été soumis à une dénaturation thermique (10 minutes à 59°C). On a ensuite comparé l'activité induite par la protéine non dénaturée à l'activité induite par cette même protéine dénaturée et on en a déduit l'activité résiduelle après dénaturation thermique pour la protéine étudiée (Activité résiduelle = Activité dénaturée/ Activité non dénaturée * 100). Ces valeurs représentent une moyenne de 5 manipulations différentes (chacune en duplicata au minimum). Les barres d'erreur présentées sont calculées sur la formule de l'erreur standard sur la moyenne (s.e.m).

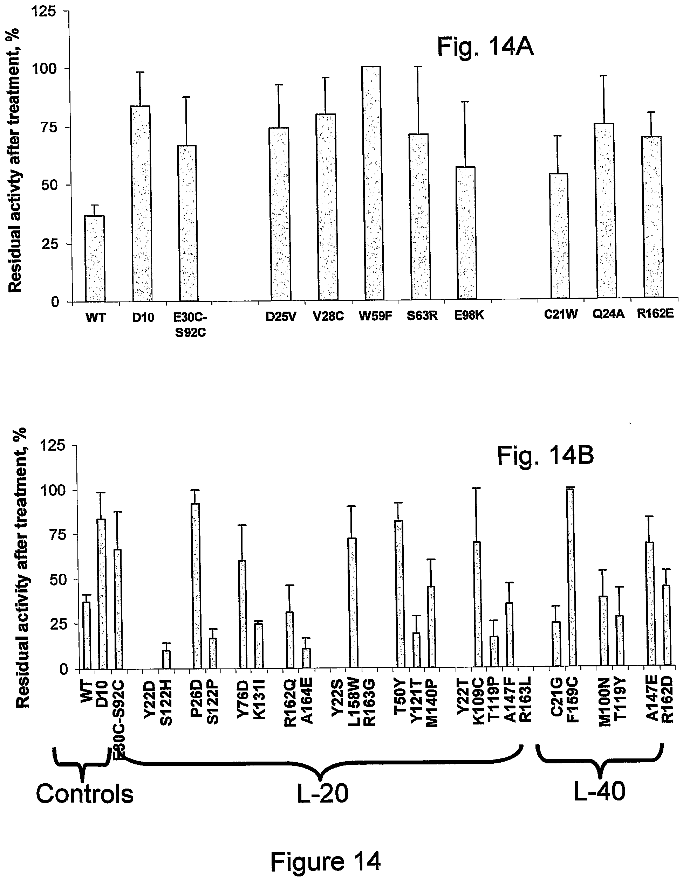

Les résultats de thermostabilité et d'activité relative à L'IFNγ non muté sont présentés en figure 14 : Certains des mutants présentent une activité résiduelle très supérieure à celle de l'IFN de type sauvage. Notamment, toutes les mutations identifiées sur des mutants simples lors du criblage primaire sont porteuses de ce gain d'activité résiduelle. Une partie seulement des mutations identifiées sur des mutants multiples lors du criblage primaire véhiculent cette amélioration d'activité résiduelle. Dans la plupart des cas, on peut estimer que l'amélioration de thermostabilité était véhiculée par une seule mutation parmi plusieurs dans ces mutants multiples, les autres mutations pouvant être considérées comme « entraînées » (et neutres sur la thermostabilité). Une exception notable est le cas du double mutant A147E R162D : chacune des deux mutations est associée à un gain d'activité résiduelle.

En conclusion : la sélection de mutants de l'IFNγ plus thermostables a été rapidement obtenue à partir d'une banque de mutants aléatoires sans qu'il soit besoin de procéder à aucune activité de criblage à haut-débit.

### Légendes des figures.

Dans toutes les figures, la séquence codant la protéine de fusion *Pᵢ-Π* est représentée de telle sorte que *Pᵢ* est en amont. Ceci correspond à la configuration préférée. Dans un mode de réalisation particulier, l'orientation contraire est utilisée, avec Π en amont. Eventuellement, une séquence codant un espaceur peptidique d'une dizaine d'acides aminés est insérée entre la séquence codant *Pᵢ* et la séquence codant *Pᵢ* ; pour simplifier cette séquence n'est pas représentée.
**Figure 1** **:** exemple de plasmide pouvant être utilisé. Cas où la diversité génétique de la banque qui sera soumise à sélection est créée après le clonage du gène d'intérêt (par exemple, par la technique Massive Mutagenesis^{®}). 1. Promoteur d'expression dans l'hôte extrêmophile ; 2. Promoteur d'expression dans *E. coli ;* 3. Origine de réplication de l'hôte extrêmophile ; 4. Origine de réplication de *E. coli.*
**Figure 2** **:** exemple de plasmide pouvant être utilisé. Cas où la banque de variants du gène d'intérêt est directement clonée avant la sélection par le procédé selon l'invention. Cette banque est créée, par exemple, par recombinaison *in vitro,* par une technique du metagenome, ou par la technique Massive Mutagenesis^{®}. 1. Promoteur d'expression dans l'hôte extrêmophile ; 2. Promoteur d'expression dans *E. coli ;* 3. Origine de réplication de l'hôte extrêmophile ; 4. Origine de réplication de *E. coli.*
**Figure 3** **:** mutagenèse dirigée sur le gène de la protéine d'intérêt puis insertion dans un plasmide d'expression en fusion avec le gène de la protéine rapporteur.
**Figure 4** **:** insertion du gène de la protéine d'intérêt en fusion avec le gène de la protéine rapporteur puis mutagenèse dirigée sur le gène de la protéine d'intérêt, par exemple par la technique Massive Mutagenesis^{®} aboutissant à une banque de variants de la protéine d'intérêt dans un vecteur d'expression de tel sorte que la protéine soit exprimée sous forme d'une protéine de fusion avec la protéine rapporteur.
**Figure 5** **:** transformation de bactéries extrêmophiles compétentes par la banque de variants contenant le gène muté de la protéine d'intérêt en fusion avec le gène de la protéine rapporteur (par exemple : un gène de résistance à un antibiotique) puis sélection sur milieu sélectif solide.
**Figure 6** **:** transformation de bactéries extrêmophiles compétentes par la banque de variants contenant le gène muté de la protéine d'intérêt en fusion avec le gène de la protéine rapporteur puis sélection en milieu sélectif liquide.

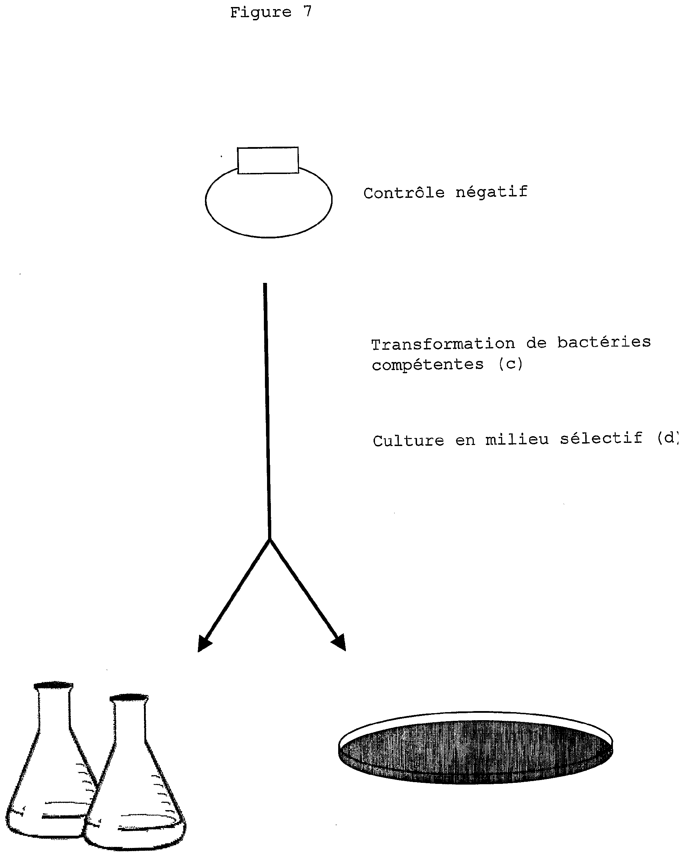

**Figure 7** **:** Contrôle négatif en milieu liquide ou solide. La transformation de bactéries extrêmophiles par du vecteur non muté ne permet pas leur croissance dans les condition retenues pour la sélection.
**Figure 8** **:** transformation de bactéries compétentes extrêmophiles avec la banque de variants contenant le gène muté de la protéine d'intérêt en fusion avec le gène de la protéine rapporteur puis visualisation de la protéine rapporteur en milieu solide.
**Figure 9** **:** transformation de bactéries compétentes par la banque de variants contenant le gène muté de la protéine d'intérêt en fusion avec le gène de la protéine rapporteur puis tri par FACS (fluorescence activated cell sorter).
**Figure 10** **:** vecteurs utilisés pour sélectionner des mutants thermostables dans *T*. *Thermophilus.* Le vecteur pNCK contient une origine de réplication de *T. Thermophilus,* une origine de réplication de *E. Coli,* un gène de résistance à l'ampicilline placé sous le contrôle d'un promoteur propre à *E. Coli,* ainsi qu'un mutant thermostable de la kanamycine nucleotidyl transférase, placé sous le contrôle d'un promoteur fonctionnant en T. Thermophilus. Ce plasmide contient en outre un peptide de liaison à son extrémité 5'. Le vecteur pNCK- est identique à pNCK, sauf qu'il ne contient pas de peptide de liaison à son extrémité 5'. Dans le vecteur pNCK-IFNγ, on a cloné la phase codante de l'IFNg en amont du peptide de liaison, en prenant soin de conserver la phase de lecture.
**Figure 11** **:** mise en place du système en *T. Thermophilus.* Les différents vecteurs ont été transformés dans *T. Thermophilus* en utilisant la compétence naturelle de cet organisme. Les bactéries transformées ont ensuite été spottées sur une boite de petri contenant diverses concentrations de kanamycine, et incubées à différentes températures.
**Figure 12** **:** Confirmation en *T. Thermophilus* de la thermorésistance accrue de certains mutants. L'ADN des mutants retenus lors de la première sélection a été préparé et retransformé en *T. Thermophilus.* Les bactéries transformées ont ensuite été étalées dans des conditions de basse stringeance (70°C, 20 μg/ml kanamycine), de haute stringeance (70°C, 40 μg/ml kanamycine), ou de stringeance nulle (60°C, 20 μg/ml kanamycine). Le nombre de colonies a été établi dans chacun de ces cas, et les ratios (basse stringeance/stringeance nulle) et (haute stringeance/stringeance nulle) ont été calculés.
**Figure 13** **:** Mesure de l'activité fonctionnelle des mutants en comparaison de l'IFNγ de type sauvage. Certains mutants ont une activité très inférieure à celle de l'IFNγ de type sauvage. D'autres ont une activité conservée ou partiellement conservée. Fig. 13A : mutants simples introduits dans le vecteur d'expression eucaryote à partir des mutations identifiés sur les mutants simples obtenus lors de la sélection primaire. Fig. 13B : mutants introduits dans le vecteur d'expression eucaryote à partir des mutations identifiées sur les mutants multiples obtenus lors de la sélection primaire.
**Figure 14** **:** Confirmation en cellules mammifère de la résistance accrue de certains mutants. Les activités en cellules mammifères ont été réalisées avant et après un traitement à 59°C pendant 10 minutes. Le ratio obtenu pour l'IFNγ de type sauvage est d'environ 37%. Les ratios supérieurs correspondent à des mutants ayant une thermostabilité accrue. Fig. 14A : mutants simples introduits dans le vecteur d'expression eucaryote à partir des mutations identifiés sur les mutants simples obtenus lors de la sélection primaire. Fig. 14B : mutants introduits dans le vecteur d'expression eucaryote à partir des mutations identifiées sur les mutants multiples obtenus lors de la sélection primaire.

## Revendications

1. Procédé de sélection de variants thermostables d'une protéine d'intérêt **caractérisé en ce qu'**une banque de variants d'une protéine d'intérêt est exprimée dans un micro-organisme extrêmophile thermophile ou hyper-thermophile sous la forme d'une protéine de fusion avec une protéine rapporteur thermostable, et **en ce que** un ou plusieurs variants thermostables de la protéine d'intérêt sont sélectionnés à une température comprise entre 50 et 110°C à partir des micro-organismes extrêmophiles dans lesquels la protéine rapporteur thermostable est active.

2. Procédé selon la revendication 1, **caractérisé en ce que** le micro-organisme extrêmophile est une bactérie, une archéo-bactérie, ou un eucaryote.

3. Procédé selon l'une quelconque des revendications précédentes, **caractérisé en ce que** le micro-organisme est sélectionné parmi les micro-organismes thermophiles ou hyper-thermophiles suivant : *Thermus aquaticus, Thermus thermophilus, Thermotoga maritime* ou *Aquifex pyrophilus.*

4. Procédé selon l'une quelconque des revendications précédentes, **caractérisé en ce que** la protéine rapporteur thermostable est une protéine dont l'expression correcte permet la survie du micro-organisme et **en ce que** la sélection du variant thermostable de la protéine d'intérêt est opérée par survie du micro-organisme.

5. Procédé selon l'une quelconque des revendications précédentes, **caractérisé en ce que** la protéine rapporteur thermostable est une version thermostable d'une protéine de résistance à un antibiotique.

6. Procédé selon la revendication 5, **caractérisé en ce que** la protéine rapporteur thermostable est une version thermostable d'une protéine de résistance à la kanamycine ou à la bléomycine.

7. Procédé selon la revendication 6, **caractérisé en ce que** la protéine rapporteur thermostable est une kanamycine nucleotidyltransférase thermostable.

8. Procédé selon l'une quelconque des revendications 1 à 4, **caractérisé en ce que** la protéine rapporteur thermostable est un marqueur d'auxotrophie.

9. Procédé selon l'une quelconque des revendications 1 à 3, **caractérisé en ce que** la protéine rapporteur thermostable est une protéine dont l'expression correcte conduit directement ou indirectement à la transformation d'un substrat en un produit, le produit et le substrat différant par leurs couleurs, et **en ce que** la sélection du variant thermostable de la protéine d'intérêt est opérée sur la base de la couleur des micro-organismes.

10. Procédé selon la revendication 9, **caractérisé en ce que** la protéine rapporteur thermostable est une version stable dans des conditions physico-chimiques non standard de la béta-galactosidase, lesdites conditions physico-chimiques non standard étant une température comprise entre 50°C et 110°C.

11. Procédé selon l'une quelconque des revendications 1 à 3, **caractérisé en ce que** la protéine rapporteur thermostable est fluorescente ou luminescente et **en ce que** la sélection du variant thermostable de la protéine d'intérêt est opérée par détection de la fluorescence ou luminescence.

12. Procédé selon la revendication 11, **caractérisé en ce que** la protéine rapporteur thermostable n'est pas fluorescente ou luminescente dans les conditions physico-chimiques non standard où elle est produite mais devient fluorescente lorsqu'on la place dans des conditions physico-chimiques standard, lesdites conditions physico-chimiques non standard étant une température comprise entre 50°C et 110°C et lesdites conditions physico-chimiques standard étant une température ambiante.

13. Procédé selon l'une quelconque des revendications 11 à 12, **caractérisé en ce que** la protéine rapporteur est un variant thermostable de la GFP (green fluorescent protein).

14. Procédé selon l'une quelconque des revendications 10 à 13, **caractérisé en ce que** la sélection est effectuée en utilisant un FACS ou par simple sélection visuelle de colonies bactériennes.

15. Procédé selon l'une quelconque des revendications précédentes, **caractérisé en ce que** la protéine de fusion comprend un espaceur peptidique.

16. Procédé selon l'une quelconque des revendications précédentes, **caractérisé en ce que** la protéine d'intérêt est située en N-terminal de la protéine rapporteur thermostable dans la protéine de fusion.

17. Procédé selon l'une quelconque des revendications 1 à 15, **caractérisé en ce que** la protéine d'intérêt est située en C-terminal de la protéine rapporteur thermostable dans la protéine de fusion.

18. Procédé selon l'une quelconque des revendications précédentes, **caractérisé en ce que** la banque de variants de la protéine d'intérêt est générée par une technique de mutagenèse quelconque, de préférence sélectionnée parmi le groupe suivant : la technologie Massive Mutagenesis^{®}, la mutagenèse aléatoire, la mutagenèse dirigée, la mutagenèse à saturation, la mutagenèse par élongation, et la mutagenèse *in vivo,* une méthode de recombinaison génétique *in vivo* ou *in vitro,* ou une combinaison de celles-ci.

19. Procédé selon la revendication 18, **caractérisé en ce que** la banque de variants de la protéine d'intérêt est créée par la technologie Massive Mutagenesis^{®}.

20. Procédé selon la revendication 18, **caractérisé en ce que** la banque de variants de la protéine d'intérêt est créée par recombinaison *in vitro* circulaire de plusieurs gènes naturels ou synthétiques codant la protéine d'intérêt.

21. Procédé selon l'une quelconque des revendications 1 à 17, **caractérisé en ce que** la banque de variants de la protéine d'intérêt est créée par clonage direct d'acides nucléiques tirés d'une source environnementale par utilisation des techniques du metagénome.

22. Procédé selon l'une quelconque des revendications 1-5, 16 et 18-19, **caractérisé en ce que** le micro-organisme est thermophile ou hyper-thermophile, la protéine rapporteur est une protéine thermostable conférant la résistance à un antibiotique, la protéine d'intérêt est située en N-terminal de la protéine rapporteur dans la protéine de fusion, et la banque de variants de la protéine d'intérêt est créée par la technologie Massive Mutagenesis^{®}.

23. Procédé selon la revendication 22, **caractérisé en ce que** le micro-organisme est T. Thermophilus, que la protéine rapporteur est une kanamycine nucleotidyltransférase thermostable, et que la protéine de fusion comprend un espaceur peptidique.

24. Protéine de fusion comprenant une protéine d'intérêt ou un variant de celle-ci et une version thermostable d'une protéine conférant la résistance à un antibiotique.

25. Protéine de fusion selon la revendication 24, **caractérisée en ce que** la protéine conférant la résistance à un antibiotique est la kanamycine nucleotidyltransférase ou la protéine de résistance à la bléomycine.

26. Acide nucléique ou vecteur d'expression codant la protéine de fusion selon la revendication 24 ou 25.

27. Vecteur selon la revendication 26, **caractérisé en ce que** le vecteur comprend les éléments nécessaires à la réplication dans un micro-organisme extrêmophile.

28. Vecteur selon la revendication 27, **caractérisé en ce que** le vecteur comprend en outre une origine de réplication pour un autre micro-organisme couramment utilisé en laboratoire, par exemple *E. Coli,* et/ou un autre gène de résistance à un antibiotique.

## Patentansprüche

1. Verfahren zur Selektion thermostabiler Varianten eines Proteins von Interesse, **dadurch gekennzeichnet, dass** eine Bank von Varianten eines Proteins von Interesse in einem thermophilen oder hyperthermophilen extremophilen Mikroorganismus in Form eines Fusionsproteins mit einem thermostabilen Reporterprotein exprimiert wird und dass eine oder mehrere thermostabile Varianten des Proteins von Interesse bei einer Temperatur zwischen 50 und 110°C von den extremophilen Mikroorganismen selektiert werden, in denen das thermostabile Reporterprotein aktiv ist.

2. Verfahren gemäß Anspruch 1, **dadurch gekennzeichnet, dass** der extremophile Mikroorganismus ein Bakterium, ein Archaebakterium oder ein Eukaryot ist.

3. Verfahren gemäß einem der vorhergehenden Ansprüche, **dadurch gekennzeichnet, dass** der Mikroorganismus aus folgenden thermophilen oder hyperthermophilen Mikroorganismen ausgewählt ist: *Thermus aquaticus, Thermus thermophilus, Thermotoga maritime* oder *Aquifex pyrophilus.*

4. Verfahren gemäß einem der vorhergehenden Ansprüche, **dadurch gekennzeichnet, dass** das thermostabile Reporterprotein ein Protein ist, dessen korrekte Expression das Überleben des Mikroorganismus ermöglicht, und dass die Selektion der thermostabilen Variante des Proteins von Interesse mithilfe des Überlebens des Mikroorganismus durchgeführt wird.

5. Verfahren gemäß einem der vorhergehenden Ansprüche, **dadurch gekennzeichnet, dass** das thermostabile Reporterprotein eine thermostabile Version eines Resistenzproteins gegen ein Antibiotikum ist.

6. Verfahren gemäß Anspruch 5, **dadurch gekennzeichnet, dass** das thermostabile Reporterprotein eine thermostabile Version eines Resistenzproteins gegen Kanamycin oder Bleomycin ist.

7. Verfahren gemäß Anspruch 6, **dadurch gekennzeichnet, dass** das thermostabile Reporterprotein eine thermostabile Kanamycin-Nukleotidyltransferase ist.

8. Verfahren gemäß einem der Ansprüche 1 bis 4, **dadurch gekennzeichnet, dass** das thermostabile Reporterprotein ein Auxothrophie-Marker ist.

9. Verfahren gemäß einem der Ansprüche 1 bis 3, **dadurch gekennzeichnet, dass** das thermostabile Reporterprotein ein Protein ist, dessen korrekte Expression direkt oder indirekt zur Umwandlung eines Substrats in ein Produkt führt, wobei das Produkt und das Substrat sich in ihren Farben unterscheiden, und dass die Selektion der thermostabilen Variante des Proteins von Interesse auf Basis der Farbe der Mikroorganismen durchgeführt wird.

10. Verfahren gemäß Anspruch 9, **dadurch gekennzeichnet, dass** das thermostabile Reporterprotein eine stabile Version der beta-Galactosidase unter physiko-chemischen Nicht-Standardbedingungen ist, wobei besagte physiko-chemische Nicht-Standardbedingungen eine Temperatur zwischen 50°C und 110°C bedeuten.

11. Verfahren gemäß einem der Ansprüche 1 bis 3, **dadurch gekennzeichnet, dass** das thermostabile Reporterprotein fluoreszierend oder lumineszierend ist und dass die Selektion der thermostabilen Variante des Proteins von Interesse mittels Lumineszenz- oder Fluoreszenz-Detektion durchgeführt wird.

12. Verfahren gemäß Anspruch 11, **dadurch gekennzeichnet, dass** das thermostabile Reporterprotein weder fluoreszierend noch lumineszierend unter den physiko-chemischen Nicht-Standardbedingungen ist, unter denen es produziert wird, aber fluoreszierend wird, wenn man es unter physiko-chemische Standardbedingungen bringt, wobei besagte physiko-chemische Nicht-Standardbedingungen eine Temperatur zwischen 50°C und 110°C bedeuten und besagte physiko-chemische Standardbedingungen Raumtemperatur bedeuten.

13. Verfahren gemäß einem der Ansprüche 11 bis 12, **dadurch gekennzeichnet, dass** das Reporterprotein eine thermostabile Variante von GFP (green fluorescent protein) ist.

14. Verfahren gemäß einem der Ansprüche 10 bis 13, **dadurch gekennzeichnet, dass** die Selektion unter Verwendung eines FACS oder durch einfache visuelle Selektion von Bakterienkolonien durchgeführt wird.

15. Verfahren gemäß einem der vorhergehenden Ansprüche, **dadurch gekennzeichnet, dass** das Fusionsprotein einen Peptid-Spacer umfasst.

16. Verfahren gemäß einem der vorhergehenden Ansprüche, **dadurch gekennzeichnet, dass** das Protein von Interesse am N-Terminus des thermostabilen Reporterproteins in dem Fusionsprotein liegt.

17. Verfahren gemäß einem der Ansprüche 1 bis 15, **dadurch gekennzeichnet, dass** das Protein von Interesse am C-Terminus des thermostabilen Reporterproteins in dem Fusionsprotein liegt.

18. Verfahren gemäß einem der vorhergehenden Ansprüche, **dadurch gekennzeichnet, dass** die Bank von Varianten des Proteins von Interesse mithilfe einer beliebigen Mutagenese-Technik generiert wird, die vorzugsweise aus der folgenden Gruppe ausgewählt ist: der Massive Mutagenesis^{®} Technologie, der zufälligen Mutagenese, der gerichteten Mutagenese, der Sättigungsmutagenese, der Elongationsmutagenese und der *in vivo* Mutagenese, einem *in vivo* oder *in vitro* genetischen Rekombinationsverfahren oder einer Kombination davon.

19. Verfahren gemäß Anspruch 18, **dadurch gekennzeichnet, dass** die Bank von Varianten des Proteins von Interesse mithilfe der Massive Mutagenesis^{®} Technologie gebildet wird.

20. Verfahren gemäß Anspruch 18, **dadurch gekennzeichnet, dass** die Bank von Varianten des Proteins von Interesse mittels zirkulärer *in vitro* Rekombination mehrerer natürlicher oder synthetischer Gene, die für das Protein von Interesse codieren, gebildet wird.

21. Verfahren gemäß einem der Ansprüche 1 bis 17, **dadurch gekennzeichnet, dass** die Bank von Varianten des Proteins von Interesse mittels direkter Klonierung von Nukleinsäuren, die aus der Umwelt stammen, unter Verwendung der Metagenom-Techniken gebildet wird.

22. Verfahren gemäß einem der Ansprüche 1 bis 5, 16 und 18 bis 19, **dadurch gekennzeichnet, dass** der Mikroorganismus thermophil oder hyperthermophil ist, das Reporterprotein ein thermostabiles Protein ist, das die Resistenz gegen ein Antibiotikum verleiht, das Protein von Interesse am N-Terminus des Reporterproteins in dem Fusionsprotein liegt und die Bank von Varianten des Proteins von Interesse mithilfe der Massive Mutagenesis^{®} Technologie gebildet wird.

23. Verfahren gemäß Anspruch 22, **dadurch gekennzeichnet, dass** der Mikroorganismus T. *thermophilus* ist, dass das Reporterprotein eine thermostabile Kanamycin-Nukleotidyltransferase ist und dass das Fusionsprotein einen Peptid-Spacer umfasst.

24. Fusionsprotein, umfassend ein Protein von Interesse oder eine Variante davon und eine thermostabile Version eines Proteins, das die Resistenz gegen ein Antibiotikum verleiht.

25. Fusionsprotein gemäß Anspruch 24, **dadurch gekennzeichnet, dass** das Protein, das die Resistenz gegen ein Antibiotikum verleiht, die Kanamycin-Nukleotidyltransferase oder das Resistenzprotein gegen Bleomycin ist.

26. Nukleinsäure oder Expressionsvektor, codierend das Fusionsprotein gemäß Anspruch 24 oder 25.

27. Vektor gemäß Anspruch 26, **dadurch gekennzeichnet, dass** der Vektor die für die Replikation in einem extremophilen Mikroorganismus erforderlichen Element umfasst.

28. Vektor gemäß Anspruch 27, **dadurch gekennzeichnet, dass** der Vektor außerdem einen Replikationsursprung für einen im Labor häufig verwendeten weiteren Mikroorganismus, zum Beispiel *E. coli,* und/oder ein weiteres Resistenzgen gegen ein Antiobiotikum umfasst.

## Claims

1. A process for selecting thermostable variants of a protein of interest **characterized in that** a library of variants of a protein of interest is expressed in an thermophilic or hyper-thermophilic extremophilic microorganism as a fusion protein with a thermostable reporter protein, and **in that**, one or several thermostable variants of the protein of interest are selected at a temperature comprised between 50 and 110°C from the extremophilic microorganisms in which the thermostable reporter protein is active.

2. The process according to claim 1, **characterized in that** the extremophilic microorganism is a bacterium, an archaebacterium, or a eukaryote.

3. The process according to any one of the preceding claims, **characterized in that** the microorganism is selected from the following thermophilic or hyper-thermophilic microorganisms: *Thermus aquaticus, Thermus thermophilus, Thermotoga maritime* or *Aquifex pyrophilus.*

4. The process according to any one of the preceding claims, **characterized in that** the thermostable reporter protein is a protein whose correct expression allows the survival of the microorganism and **in that** the selection of the thermostable variant of the protein of interest is performed by the survival of the microorganism.

5. The process according to any one of the preceding claims, **characterized in that** the thermostable reporter protein is a thermostable version of a protein conferring a resistance to an antibiotic.

6. The process according to claim 5, **characterized in that** the thermostable reporter protein is a thermostable version of a kanamycin or bleomycin resistance protein.

7. The process according to claim 6, **characterized in that** the thermostable reporter protein is a thermostable kanamycin nucleotidyltransferase.

8. The process according to any one of claims 1 to 4, **characterized in that** the thermostable reporter protein is an auxotrophy marker.

9. The process according to any one of claims 1 to 3, **characterized in that** the thermostable reporter protein is a protein whose correct expression directly or indirectly leads to the transformation of a substrate into a product, said product and substrate having different colors, and **in that** the selection of the thermostable variant of the protein of interest is performed by the color of microorganisms.

10. The process according to claim 9, **characterized in that** the thermostable reporter protein is a version of beta-galactosidase which is stable in non-standard physicochemical conditions, said non-standard physico-chemical conditions being a temperature comprised between 50°C and 110°C.

11. The process according to any one of claims 1 to 3, **characterized in that** the thermostable reporter protein is fluorescent or luminescent and **in that** the selection of the thermostable variant of the protein of interest is performed by detecting fluorescence or luminescence.

12. The process according to claim 11, **characterized in that** the thermostable reporter protein is not fluorescent or luminescent in non-standard physico-chemical conditions where it is produced but becomes fluorescent when it is placed in standard physicochemical conditions, said non-standard physico-chemical conditions being a temperature comprised between 50°C and 110°C and said standard physico-chemical conditions being a room temperature.

13. The process according to any one of claims 11 to 12, **characterized in that** the reporter protein is a thermostable variant of GFP (green fluorescent protein).

14. The process according to any one of claims 10 to 13, **characterized in that** the selection is performed by using FACS or by simple visual selection of bacterial colonies.

15. The process according to any one of preceding claims, **characterized in that** the fusion protein comprises a peptide linker.

16. The process according to any one of preceding claims, **characterized in that** the protein of interest is located at the N-terminal of the thermostable reporter protein in the fusion protein.

17. The process according to any one of claims 1 to 15, **characterized in that** the protein of interest is located at the C-terminal of the thermostable reporter protein in the fusion protein.

18. The process according to any one of the preceding claims, **characterized in that** the library of variants of the protein of interest is generated by any mutagenesis technology, preferably selected from the group consisting of: Massive Mutagenesis^{®} technology, random mutagenesis, site specific mutagenesis, saturation mutagenesis, elongation mutagenesis, and *in vivo* mutagenesis, an *in vivo* or *in vitro* genetic recombination method, or a combination thereof.

19. The process according to claim 18, **characterized in that** the library of variants of the protein of interest is generated by Massive Mutagenesis^{®} technology.

20. The process according to claim 18, **characterized in that** the library of variants of the protein of interest is generated by *in vitro* circular recombination of several natural or synthetic genes encoding the protein of interest.

21. The process according to any one of claims 1 to 17, **characterized in that** the library of variants of the protein of interest is generated by direct cloning of nucleic acids from an environmental source using metagenomics technologies.

22. The process according to any one of claims 1-5, 16 and 18-19, **characterized in that** the microorganism is thermophilic or hyper-thermophilic, the reporter protein is a thermostable protein conferring a resistance to an antibiotic, the protein of interest is located at the N-terminal of the reporter protein in the fusion protein, and the library of variants of the protein of interest is generated by Massive Mutagenesis® technology.

23. The process according to claim 22, **characterized in that** the microorganism is T. thermophilus, the reporter protein is a thermostable kanamycin nucleotidyltransferase, and the fusion protein comprises a peptide linker.

24. Fusion protein comprising a protein of interest or a variant thereof and a thermostable version of a protein conferring a resistance to an antibiotic.

25. Fusion protein according to claim 24, **characterized in that** the protein conferring a resistance to an antibiotic is a kanamycin nucleotidyltransferase or a bleomycin resistance protein.

26. Nucleic acid or expression vector encoding a fusion protein according to claim 24 or 25.

27. A vector according to claim 26, **characterized in that** the vector comprises the elements necessary for replication in an extremophilic microorganism.

28. Vector according to claim 27, **characterized in that** the vector further comprises an origin of replication for another microorganism commonly used in laboratory, for example E. coli, and/or another gene for antibiotic resistance.


### SEQUENCE LISTING

<110> BIOMETHODES
<120> Méthode de sélection dans des conditions physico-chimiques non
   standard de protéines stables
<130> B0374WO
<150> FR 05 05935
   <151> 2005-06-10
<160> 8
<170> PatentIn version 3.1
<210> 1
   <211> 744
   <212> DNA
   <213> artificial sequence
<220>
   <223> variant thermorésistant du gène de résistance à la kanamycine
<220>
   <221> CDS
   <222> (1)..(744)
   <223>
<400> 1
   atg act aga gaa gaa aga atg aag att gtt cat gaa att aag gaa cga      48
   Met Thr Arg Glu Glu Arg Met Lys Ile Val His Glu Ile Lys Glu Arg
   1               5                   10                  15
   ata ttg gat aaa tat ggg gat gat gtt aag gct att ggt gtt tat ggc      96
   Ile Leu Asp Lys Tyr Gly Asp Asp Val Lys Ala Ile Gly Val Tyr Gly
               20                  25                  30
   tct ctt ggt cgt cag act gat ggg ccc tat tcg gat att gag atg atg     144
   Ser Leu Gly Arg Gln Thr Asp Gly Pro Tyr Ser Asp Ile Glu Met Met
           35                  40                  45
   tgt gtc atg tca aca gag gaa gca gag ttc agc cat gaa tgg aca acc     192
   Cys Val Met Ser Thr Glu Glu Ala Glu Phe Ser His Glu Trp Thr Thr
   50 55 60
   ggt gag tgg aag gtg gaa gtg aat ttt tat agc gaa gag att cta cta 240
   Gly Glu Trp Lys Val Glu Val Asn Phe Tyr Ser Glu Glu Ile Leu Leu
   65 70 75 80
   gat tat gca tct cag gtg gaa tca gat tgg ccg ctt aca cat ggt caa 288
   Asp Tyr Ala Ser Gln Val Glu Ser Asp Trp Pro Leu Thr His Gly Gln
   85 90 95
   ttt ttc tct att ttg ccg att tat gat tca ggt gga tac tta gag aaa 336
   Phe Phe Ser Ile Leu Pro Ile Tyr Asp Ser Gly Gly Tyr Leu Glu Lys
   100 105 110
   gtg tat caa act gct aaa tcg gta gaa gcc caa aag ttc cac gat gcg 384
   Val Tyr Gln Thr Ala Lys Ser Val Glu Ala Gln Lys Phe His Asp Ala
   115 120 125
   att tgt gcc ctt atc gta gaa gag ctg ttt gaa tat gca ggc aaa tgg 432
   Ile Cys Ala Leu Ile Val Glu Glu Leu Phe Glu Tyr Ala Gly Lys Trp
   130 135 140
   cgt aat att cgt gtg caa gga ccg aca aca ttt cta cca tcc ttg act 480
   Arg Asn Ile Arg Val Gln Gly Pro Thr Thr Phe Leu Pro Ser Leu Thr
   145 150 155 160
   gta cag gta gca atg gca ggt gcc atg ttg att ggt ctg cat cat cgc 528
   Val Gln Val Ala Met Ala Gly Ala Met Leu Ile Gly Leu His His Arg
   165 170 175
   atc tgt tat acg acg agc gct tcg ctc tta act gaa gca gtt aag caa 576
   Ile Cys Tyr Thr Thr Ser Ala Ser Leu Leu Thr Glu Ala Val Lys Gln
   180 185 190
   tca gat ctt cct tca ggt tat gac cat ctg tgc cag ttc gta atg tct 624
   Ser Asp Leu Pro Ser Gly Tyr Asp His Leu Cys Gln Phe Val Met Ser
   195 200 205
   ggt caa ctt tcc gac tct gag aaa ctt ctg gaa tcg cta gag aat ttc 672
   Gly Gln Leu Ser Asp Ser Glu Lys Leu Leu Glu Ser Leu Glu Asn Phe
   210 215 220
   tgg aat ggg att cag gag tgg aca gaa cga cac gga tat ata gtg gat 720
   Trp Asn Gly Ile Gln Glu Trp Thr Glu Arg His Gly Tyr Ile Val Asp
   225 230 235 240
   gtg tca aaa cgc ata cca ttt tga 744
   Val Ser Lys Arg Ile Pro Phe
   245
<210> 2
   <211> 247
   <212> PRT
   <213> artificial sequence
<220>
   <223> variant thermorésistant du gène de résistance à la kanamycine
<400> 2
   Met Thr Arg Glu Glu Arg Met Lys Ile Val His Glu Ile Lys Glu Arg
   1 5 10 15
   Ile Leu Asp Lys Tyr Gly Asp Asp Val Lys Ala Ile Gly Val Tyr Gly
   20 25 30
   Ser Leu Gly Arg Gln Thr Asp Gly Pro Tyr Ser Asp Ile Glu Met Met
   35 40 45
   Cys Val Met Ser Thr Glu Glu Ala Glu Phe Ser His Glu Trp Thr Thr
   50 55 60
   Gly Glu Trp Lys Val Glu Val Asn Phe Tyr Ser Glu Glu Ile Leu Leu
   65 70 75 80
   Asp Tyr Ala Ser Gln Val Glu Ser Asp Trp Pro Leu Thr His Gly Gln
   85 90 95
   Phe Phe Ser Ile Leu Pro Ile Tyr Asp Ser Gly Gly Tyr Leu Glu Lys
   100 105 110
   Val Tyr Gln Thr Ala Lys Ser Val Glu Ala Gln Lys Phe His Asp Ala
   115 120 125
   Ile Cys Ala Leu Ile Val Glu Glu Leu Phe Glu Tyr Ala Gly Lys Trp
   130 135 140
   Arg Asn Ile Arg Val Gln Gly Pro Thr Thr Phe Leu Pro Ser Leu Thr
   145 150 155 160
   Val Gln Val Ala Met Ala Gly Ala Met Leu Ile Gly Leu His His Arg
   165 170 175
   Ile Cys Tyr Thr Thr Ser Ala Ser Leu Leu Thr Glu Ala Val Lys Gln
   180 185 190
   Ser Asp Leu Pro Ser Gly Tyr Asp His Leu Cys Gln Phe Val Met Ser
   195 200 205
   Gly Gln Leu Ser Asp Ser Glu Lys Leu Leu Glu Ser Leu Glu Asn Phe
   210 215 220
   Trp Asn Gly Ile Gln Glu Trp Thr Glu Arg His Gly Tyr Ile Val Asp
   225 230 235 240
   Val Ser Lys Arg Ile Pro Phe
   245
<210> 3
   <211> 375
   <212> DNA
   <213> artificial sequence
<220>
   <223> variant thermorésistant du gène de résistance à la bléomycine
<220>
   <221> CDS
   <222> (1)..(375)
   <223>
<400> 3
   atg gct aaa ctt act tct gct gtt cct gtt ctt aca gct aga gat gta       48
   Met Ala Lys Leu Thr Ser Ala Val Pro Val Leu Thr Ala Arg Asp Val
   1               5                   10                  15
   gca gaa gct gtt gaa ttt tgg act gat aga ttg ggt ttt tct agg gtt       96
   Ala Glu Ala Val Glu Phe Trp Thr Asp Arg Leu Gly Phe Ser Arg Val
               20                  25                  30
   ttc gtg gaa gac gat ttt gca ggt gtt gtt aga gat gat gta aca ctt      144
   Phe Val Glu Asp Asp Phe Ala Gly Val Val Arg Asp Asp Val Thr Leu
           35                  40                  45
   ttt att tca gct gtg caa gat caa gtt gtt cca gat aat act caa gca      192
   Phe Ile Ser Ala Val Gln Asp Gln Val Val Pro Asp Asn Thr Gln Ala
       50                  55                  60
   tgg gta tgg gtt aga ggt ttg gat gag ctt tat gct gaa tgg tct gaa      240
   Trp Val Trp Val Arg Gly Leu Asp Glu Leu Tyr Ala Glu Trp Ser Glu
   65                  70                  75                  80
   gtt gtg tct aca aat ttt agg gat gca agt ggt cct gct atg act gag      288
   Val Val Ser Thr Asn Phe Arg Asp Ala Ser Gly Pro Ala Met Thr Glu
                   85                  90                  95
   att gtt gaa caa cct tgg ggt aga gaa ttt gca ctt aga gat cct gct      336
   Ile Val Glu Gln Pro Trp Gly Arg Glu Phe Ala Leu Arg Asp Pro Ala
               100                 105                 110
   ggt aat tgt gtt cat ttt gtt gca gag gaa caa gat tga                  375
   Gly Asn Cys Val His Phe Val Ala Glu Glu Gln Asp
           115                 120
<210> 4
   <211> 124
   <212> PRT
   <213> artificial sequence
<220>
   <223> variant thermorésistant du gène de résistance à la bléomycine
<400> 4
   Met Ala Lys Leu Thr Ser Ala Val Pro Val Leu Thr Ala Arg Asp Val
   1               5                   10                  15
   Ala Glu Ala Val Glu Phe Trp Thr Asp Arg Leu Gly Phe Ser Arg Val
                   20                  25                  30
   Phe Val Glu Asp Asp Phe Ala Gly Val Val Arg Asp Asp Val Thr Leu
           35                  40                  45
   Phe Ile Ser Ala Val Gln Asp Gln Val Val Pro Asp Asn Thr Gln Ala
       50                  55                  60
   Trp Val Trp Val Arg Gly Leu Asp Glu Leu Tyr Ala Glu Trp Ser Glu
   65                  70                  75                  80
   Val Val Ser Thr Asn Phe Arg Asp Ala Ser Gly Pro Ala Met Thr Glu
                   85                  90                  95
   Ile Val Glu Gln Pro Trp Gly Arg Glu Phe Ala Leu Arg Asp Pro Ala
               100                 105                 110
   Gly Asn Cys Val His Phe Val Ala Glu Glu Gln Asp
           115                 120
<210> 5
   <211> 441
   <212> DNA
   <213> Homo sapiens
<220>
   <221> CDS
   <222> (1)..(441)
   <223>
<400> 5
   tgt tac tgc cag gac cca tat gta caa gaa gca gaa aac ctt aag aaa      48
   Cys Tyr Cys Gln Asp Pro Tyr Val Gln Glu Ala Glu Asn Leu Lys Lys
   1               5                   10                  15
   tat ttt aat gca ggt cat tca gat gta gcg gat aat gga act ctt ttc      96
   Tyr Phe Asn Ala Gly His Ser Asp Val Ala Asp Asn Gly Thr Leu Phe
               20                  25                  30
   tta ggc att ttg aag aat tgg aaa gag gag agt gac aga aaa ata atg     144
   Leu Gly Ile Leu Lys Asn Trp Lys Glu Glu Ser Asp Arg Lys Ile Met
           35                  40                  45
   cag agc caa att gtc tcc ttt tac ttc aaa ctt ttt aaa aac ttt aaa     192
   Gln Ser Gln Ile Val Ser Phe Tyr Phe Lys Leu Phe Lys Asn Phe Lys
       50                  55                  60
   gat gac cag agc atc caa aag agt gtg gag acc atc aag gaa gac atg     240
   Asp Asp Gln Ser Ile Gln Lys Ser Val Glu Thr Ile Lys Glu Asp Met
   65                  70                  75                  80
   aat gtc aag ttt ttc aat agc aac aaa aag aaa cga gat gac ttc gaa     288
   Asn Val Lys Phe Phe Asn Ser Asn Lys Lys Lys Arg Asp Asp Phe Glu
                   85                  90                  95
   aag ctg act aat tat tcg gta act gac ttg aat gtc caa cgc aaa gca       336
   Lys Leu Thr Asn Tyr Ser Val Thr Asp Leu Asn Val Gln Arg Lys Ala
               100                 105                 110
   ata cat gaa ctc atc caa gtg atg gct gaa ctg tcg cca gca gct aaa       384
   Ile His Glu Leu Ile Gln Val Met Ala Glu Leu Ser Pro Ala Ala Lys
           115                 120                 125
   aca ggg aag cga aaa agg agt cag atg ctg ttt cga ggt cga aga gca       432
   Thr Gly Lys Arg Lys Arg Ser Gln Met Leu Phe Arg Gly Arg Arg Ala
       130                 135                 140
   tcc cag taa                                                           441
   Ser Gln
   145
<210> 6
   <211> 146
   <212> PRT
   <213> Homo sapiens
<400> 6
   Cys Tyr Cys Gln Asp Pro Tyr Val Gln Glu Ala Glu Asn Leu Lys Lys
   1               5                   10                  15
   Tyr Phe Asn Ala Gly His Ser Asp Val Ala Asp Asn Gly Thr Leu Phe
               20                  25                  30
   Leu Gly Ile Leu Lys Asn Trp Lys Glu Glu Ser Asp Arg Lys Ile Met
           35                  40                  45
   Gln Ser Gln Ile Val Ser Phe Tyr Phe Lys Leu Phe Lys Asn Phe Lys
       50                  55                  60
   Asp Asp Gln Ser Ile Gln Lys Ser Val Glu Thr Ile Lys Glu Asp Met
   65                  70                  75                  80
   Asn Val Lys Phe Phe Asn Ser Asn Lys Lys Lys Arg Asp Asp Phe Glu
                   85                  90                  95
   Lys Leu Thr Asn Tyr Ser Val Thr Asp Leu Asn Val Gln Arg Lys Ala
               100                 105                 110
   Ile His Glu Leu Ile Gln Val Met Ala Glu Leu Ser Pro Ala Ala Lys
           115                 120                 125
   Thr Gly Lys Arg Lys Arg Ser Gln Met Leu Phe Arg Gly Arg Arg Ala
       130                 135                 140
   Ser Gln
   145
<210> 7
   <211> 27
   <212> DNA
   <213> artificial sequence
<220>
   <223> linker
<220>
   <221> CDS
   <222> (1)..(27)
   <223>
<400> 7
   gcg gcc gca gga agc tct ggt tcc atc                              27
   Ala Ala Ala Gly Ser Ser Gly Ser Ile
   1               5
<210> 8
   <211> 9
   <212> PRT
   <213> artificial sequence
<220>
   <223> linker
<400> 8
   Ala Ala Ala Gly Ser Ser Gly Ser Ile
   1               5